# EUROPEAN PATENT APPLICATION

(11) **EP 3 211 421 A1**
(43) Date of publication of application: **30.08.2017**
(21) Application number: 16206438.0
(22) Date of filing: 09.09.2011
(51) Int. Cl.: G01N 33/574, G01N 33/68, C12Q 1/68

(54) **COMBINATION METHODS OF DIAGNOSING CANCER IN A PATIENT**

(30) Priority: 09.09.2010 US 381130 P; 20.04.2011 US 201161477597 P
(62) Divisional of application: 11824200.7
(71) Applicant: Traxxsson, LLC, Saint Louis, MO 63108 (US)
(72) Inventor: PUSKAS, Robert, St. Louis, MO 63134-3115 (US); HELD, Douglas, St. Louis, MO 63108 (US)
(74) Representative: Smaggasgale, Gillian Helen

(57) **Abstract**

The present disclosure relates to methods for determining the presence, activity, and/or concentrations of certain cancer biomarkers and their use in determining the presence of cancer.

## Description

### FIELD OF THE INVENTION

The present invention generally relates to medical diagnostics and to methods, kits and assays for the diagnosing or otherwise determining the presence of cancer in a patient. More particularly, the present invention involves methods for determining the presence, activity, and/or concentrations of certain indicators and their use in determining the presence of cancer.

### BACKGROUND OF THE INVENTION

For the most part, conventional cancer screening assays are limited to individual tests for cancer located in a specific organ. Such tests include, for example, mammograms for breast cancer, colonoscopies for colorectal cancer, the PSA test for prostate cancer, and Pap smears for cervical cancer. However, these cancers account for only about 25% of the cancer cases detected in the United States each year. Several other types of cancer are detected only when physical symptoms appear. Consequently, cancer in many patients is detected at a late stage when mortality is greatly increased [120].

An estimated 1.4 million cases of cancer will be diagnosed in the U.S. in 2010 [121]. As the population ages, the number of cancer cases is expected to increase by 19% [122-123]. Approximately 10.8 million people alive today have, or have had, diagnosed cancer [124]. Over 20 million individuals will be screened for breast or prostate cancer this year.

Although various tests have been developed to detect cancer in specific organs, in many cases these tests are not routinely used to screen for other types of cancers, in part, because of the low cost-effectiveness of such screening.

### SUMMARY OF THE DISCLOSURE

Among the various aspects of the present invention is the provision of a method of detecting cancer in a subject.

Briefly, therefore, the present invention is directed to a method of characterizing a carcinoma in a subject, the method comprising the steps of: in a first assay, assaying a sample of a bodily fluid derived from the subject for activity of a first biomarker, the first biomarker being specific for carcinoma but not organ-specific, and in a second assay, assaying a sample derived from the subject for a second biomarker, the second biomarker being specific for cancer of an organ and other than extracellular PKA.

In one embodiment, for example, the first assay is an assay for PKA (cAMP-dependent protein kinase A) activity, anti-PKA, fibrin, fibrin derivatives or PCNA (caPCNA; proliferating cell nuclear antigen); more preferably in this embodiment, the first biomarker is extracellular PKA. In various embodiments, the first assay comprises preparing a reaction mixture comprising a sample of a bodily fluid derived from a subject, a PKA peptide substrate, a phosphorylation agent; incubating the prepared mixture; detecting phosphorylated substrate formed in the incubated mixture; and comparing the amount of phosphorylated substrate formed in the assay with a reference value, the reference value being the amount of phosphorylated substrate formed in a mixture under equivalent redox conditions for a sample of bodily fluid derived from a population of normal subjects of the same species. The sample may be, for example, a previously unfrozen sample. Alternatively, the sample may be a thawed, previously frozen sample.

Other objects and features will be in part apparent and in part pointed out hereinafter.

### BRIEF DESCRIPTION OF THE DRAWINGS

Fig. 1 is graph depicting the effect of oxidation reduction potential (mV) upon the ratio of apparent PKA activity (cancer subjects/normal subjects) as more fully described in Example 1.
Fig. 2 is graph depicting the effect of oxidation reduction potential (mV) upon the ratio of apparent PKA activity (cancer subjects/normal subjects) as more fully described in Example 2.
Fig. 3 is graph depicting PKA activity levels for samples with NaF (phosphatase inhibitor) in the reaction mixture as more fully described in Example 3.
Fig. 4 is graph depicting oxidation reduction potential and ratios of Cancer/Normal PKA activity with NaF (phosphatase inhibitor) in the reaction mixture as more fully described in Example 3.

### ABBREVIATIONS AND DEFINITIONS

The following definitions and methods are provided to better define the present invention and to guide those of ordinary skill in the art in the practice of the present invention. Unless otherwise noted, terms are to be understood according to conventional usage by those of ordinary skill in the relevant art.

As used herein, "extracellular PKA" means cAMP-dependent protein kinase A found in bodily fluids outside of bodily cells.

As used herein, "normal subject" or "normal individual" means a subject or individual not known to be afflicted with, or suspected of being afflicted with, a carcinoma.

### DETAILED DESCRIPTION

One aspect of the present invention is directed to novel methods and kits for diagnosing the presence of cancer in an animal, most preferably a human patient. The cancers to be tested include, but are not limited to, bladder cancer, bone cancer, brain cancer, breast cancer, cervical cancer, colon cancer, esophageal cancer, gastric cancer, glioma, head and neck cancer, kidney cancer, leukemia (e.g., acute myeloid leukemia (AML)), liver cancer, lung cancer, lymphoma, melanoma, mesothelioma, medulloblastoma, ovarian cancer, pancreatic cancer, prostate cancer, rectal cancer, skin cancer, testicular cancer, tracheal cancer, and vulvar cancer.

A method is described for determining the presence of cancer in a patient consisting of measuring the activity or presence of a general cancer indicator in a patient sample and determining that the activity or amount of the general cancer indicator is present at levels that are higher or lower than that of a control sample or control population. In general, the method involves assaying a sample of a bodily fluid derived from the patient for activity of a first biomarker in a first assay, and assaying a sample derived from the patient for a second biomarker in a second assay. The assays for the activity or presence of indicators that are specific for detecting cancer in a specific organ or organs are used to determine the location(s) of any cancer that may be present.

In one embodiment, the first and second assays are carried out approximately simultaneously. In another embodiment, the second assay is carried out after the results of the first assay are known. In yet another embodiment, the second assay is carried out before the results of the first assay are known. The first assay is specific for carcinoma, but not organ-specific. In a preferred embodiment, the first assay involves the measurement of activity levels of extracellular PKA as an indicator of the presence of cancer, and the second assay involves the measurement or detection of a biomarker that is specific for cancer of an organ and other than extracellular PKA.

Without being bound by any particular theory, it is believed that linking or combining an organ-specific cancer screen with a more general (e.g., not organ-specific) cancer screen can provide a cost effective means to screen for potentially all cancers, and can identify the location of over 70% of the cancers detected annually in the U.S. Based on current cancer screening rates, for instance, about 25 million individuals are screened for breast or prostate cancer annually in the U.S. As an example of the potential for cost savings derived from the methods described herein, instead of testing 25 million individuals with an organ-specific test to identify the 22,000 cases of ovarian cancer found annually, one could screen the 25 million individuals using a low cost test, and then test the 1.5 million individuals that would be expected to test positive for cancer with a group of organ-specific cancer tests including those to identify patients with ovarian cancer. Because the initial cancer screening cost is spread out over all the cases of cancer detected in a year, the cost of detecting a case of ovarian cancer could drop by 90% per case from current levels. Thus, pre-testing for cancer with a general cancer screen decreases the number of organ-specific cancer tests needed to identify the population of individuals with cancer in a specific organ. This significantly improves the cost effectiveness of identifying specific types of cancer.

In addition to providing initial cancer screenings, in some embodiments the methods of the present disclosure can be used to predict various different types of clinical outcomes. For example, the methods may be used to predict recurrence of disease state after therapy, non-recurrence of a disease state after therapy, therapy failure, short interval to disease recurrence (e.g., less than two years, or less than one year, or less than six months), short interval to metastasis in cancer (*e*.*g*., less than two years, or less than one year, or less than six months), invasiveness, non-invasiveness, likelihood of metastasis in cancer, likelihood of distant metastasis in cancer, poor survival after therapy, death after therapy, disease free survival and so forth. By way of further example, the assay methods of the present invention may be employed in a variety of different clinical situations such as, for example, in the detection of primary or secondary (metastatic) cancer, in screening for early neoplastic or early carcinogenic change in asymptomatic patients or identification of individuals "at risk" of developing cancer (*e*.*g*., breast cancer, bladder cancer, colorectal cancer or prostate cancer) in a population of asymptomatic individuals, in the detection of recurrent disease in a patient previously diagnosed as carrying tumor cells who has undergone treatment to reduce the number of tumor cells, in predicting the response of an individual with cancer to a course of anti-cancer treatment or in selection to the said treatment.

One embodiment of the present disclosure is directed to a method for diagnosing cancer or predicting cancer-therapy outcome by detecting the expression or expression levels of a general cancer biomarker (i.e., a biomarker that is not organ-specific) in a patient sample, and scoring its expression as being above a certain threshold or, more preferably, as a binary response (*e*.*g*., "yes" or "no"), and subsequently or simultaneously detecting the expression of one or more organ-specific cancer biomarkers (*e*.*g*., where the marker is from a particular pathway related to cancer), with the combined result of the two (or more) screens being indicative of a more precise, yet more cost-effective, cancer diagnosis or even a prognosis for cancer-therapy failure. This method can be used to diagnose cancer or predict cancer-therapy outcomes for a variety of cancers.

The biomarkers to be assayed within the methods of the present invention include any markers associated with cancer pathways. In one embodiment, for example, the markers may be mRNA (messenger RNA), DNA, microRNA, or protein.

In general, the methods described herein will provide for the detection of cancer and its localization to specific organs. Any sample of biological origin that can be extracted, swiped or otherwise obtained from a patient or subject and that contains a biological substance such as cells or proteins or nucleic acids may be used in connection with the methods described herein. Because the assay methods of the invention may be performed on a sample of bodily fluids taken from the patient, they may be relatively non-invasive and may be repeated as often as is thought necessary. In one preferred embodiment, it is necessary to collect only a single sample from the patient for use in the assay methods of the invention; thus, for example, the first and second assays can be carried out using separate aliquots of the same sample of a bodily fluid. Bodily fluids that may be obtained from the patient for use in the methods described herein includes peripheral blood, whole blood, serum, plasma, ascites, urine, cerebrospinal fluid (CSF), sputum, saliva, bone marrow, synovial fluid, aqueous humor, cerumen, broncheoalveolar lavage fluid, semen, prostatic fluid, cowper's fluid or pre-ejaculatory fluid, sweat, fecal matter, tears, cyst fluid, pleural and peritoneal fluid, breath condensates, nipple aspirate, lymph, chyme, chyle, bile, intestinal fluid, pus, sebum, vomit, mucosal secretion, stool water, pancreatic juice, lavage fluids from sinus cavities, or bronchopulmonary aspirates. Tissue, tumor tissue, cells, and cell cultures established from tissue may also be used, as can buchal swabs, hair follicles, and, bone marrow. The type of bodily fluid used may vary depending upon the type of cancer involved and the use to which the assay is being put. For instance, in one embodiment, the first assay involves assaying for extracellular PKA, while the second assay involves assaying for a biomarker that is specific for cancer but is other than extracellular PKA; thus, for example, the second assay is not necessary limited to extracellular biomarkers. In general, it is preferred to perform the method on samples of whole blood, serum, plasma, urine, or saliva.

In one alternative embodiment, the first assay involves assaying a sample derived from a bodily fluid for two or more general cancer biomarkers. Thus, for example, the first assay may be an assay for two or more biomarkers of the general cancer biomarkers disclosed herein. By way of further example, the first assay may be an assay for two or more biomarkers selected from PKA, fibrin, fibrin degradation products, PCNA, hTR, methylated DNA, Sga-1 m, Tadg-15, Ephrin type-A receptor 10 protein, hTR and hTERT RNA. By of further example, the first assay may be an assay for PKA and one or more biomarkers selected from fibrin, fibrin degradation products, PCNA, hTR, methylated DNA, Sga-1m, Tadg-15, Ephrin type-A receptor 10 protein, hTR and hTERT RNA. By way of further example, the first assay may be an assay for two or more biomarkers selected from PKA, non-organ specific tumor-related methylated DNA, non-organ specific tumor-related miRNA, non-organ specific tumor-related circulating nucleic acid biomarker, p53 autoantibodies, CCL2 autoantibodies, PKA autoantibodies, and prostatome autoantibodies.

In another alternative embodiment, the second assay involves assaying a sample derived from a subject for two or more organ-specific cancer biomarkers. Thus, for example, the first assay may be an assay for two or more biomarkers of the organ-specific cancer biomarkers disclosed herein. Additionally, the second assay may be an assay of a sample of a bodily fluid or may be assay (e.g., a biopsy) of a tissue sample.

In a further alternative embodiment, the first and second assays may be carried out sequentially. For example, the first assay (for one or more general cancer biomarkers) may be carried out before the second assay (for one or more organ-specific biomarkers) is carried out. By way of further example, the first assay (for one or more general cancer biomarkers) may be carried out before the second assay (for one or more organ-specific biomarkers) is carried out but both assays are carried out on separate aliquots of the same sample of a bodily fluid. By way of further example, the first assay (for one or more general cancer biomarkers) may be carried out before the second assay (for one or more organ-specific biomarkers) is carried out and the two assays are carried out on separate samples derived from the subject.

In a yet further alternative embodiment, the first and second assays may be carried out simultaneously, For example, the first assay (for one or more general cancer biomarkers) may be carried out simultaneously with the second assay (for one or more organ-specific biomarkers) and both assays are carried out on separate aliquots of the same sample of a bodily fluid. By way of further example, the first assay (for one or more general cancer biomarkers) may be carried out simultaneously with the second assay (for one or more organ-specific biomarkers), the two assays are carried out on separate samples derived from the subject, and the two assays are carried out on the same panel (or other substrate containing the necessary reagents for the two assays). By way of further example, the first assay (for one or more general cancer biomarkers) may be carried out simultaneously with the second assay (for one or more organ-specific biomarkers) and the two assays are carried out on separate samples derived from the subject.

The methods of the present invention may advantageously be used to characterize a carcinoma or otherwise assess the presence or absence of cancer in a variety of subjects. The subject may be, for example, a mammal such as bovine, avian, canine, equine, feline, ovine, porcine, or primate (including humans and non-human primates). A subject may also include mammals of importance due to being endangered, or economic importance, such as animals raised on farms for consumption by humans, or animals of social importance to humans such as animals kept as pets or in zoos. Examples of such animals include but are not limited to: cats, dogs, swine, ruminants or ungulates such as cattle, oxen, sheep, giraffes, deer, goats, bison, camels or horses. In one embodiment, the subject is a human subject. In another embodiment, the subject is bovine, avian, canine, equine, feline, ovine, porcine, or non-human primate.

The subject may have a pre-existing disease or condition, such as cancer. Alternatively, the subject may not have any known pre-existing condition. The subject may also be non-responsive to an existing or past treatment, such as a treatment for cancer.

In general, however, reference values are preferably for members of a given species. Thus, for example, PKA values for human subjects are preferably only compared to PKA values to PKA activities for a statistically significant population of human subjects under equivalent assay conditions. Similarly, PKA values for non-human subjects are preferably only compared to PKA values to PKA activities for a statistically significant population of non-human subjects of the same species under equivalent assay conditions.

### EXTRACELLULAR PKA ASSAY

As noted above, the methods described herein involve a general cancer test, preferably as an initial screen. A range of general indicators have been used to detect signs of cancer, including proteins, peptides, antibodies, autoantibodies, lipids, sugars, nucleic acids, DNA, RNA, mRNA, methylated DNA, and circulating tumor cells [1-20]. Direct or indirect tests for such indicators also can take many forms such as activity assays, determinations of modified or altered indicators, and determinations of indicator presence or quantities, to name only a few.

In general, any known general (i.e., non-organ specific) cancer screen can be employed in connection with the methods described herein, and a range of general cancer screens are known in the art. For example, various biomarker tests have been proposed as general indicators of cancer.

One particularly preferred general cancer screen is available in the form of a test for blood PKA activity. Extremely low levels of PKA activity are detected in non-reduced blood samples making accurate measurement of enzyme activity very difficult. The addition of an antioxidant to the PKA assay activates the enzyme making it much easier to measure the PKA activity and to measure differences, especially decreases, in enzyme activity. Assaying activated PKA activity in blood samples provides identification of individuals who have cancer by virtue of their low levels of activated PKA activity. This decrease in activity can be used to determine the presence of cancer in patients with breast, colorectal, lung, and prostate cancer.

In accordance with one aspect of the present invention, it has been shown that extracellular PKA may be used to characterize carcinoma in a subject. More specifically, it has been determined that extracellular PKA derived from persons unafflicted with carcinoma and extracellular PKA derived from persons afflicted with carcinoma can be differentiated, depending upon the reaction conditions. Under certain reaction conditions, extracellular PKA derived from persons unafflicted with carcinoma has a greater activity for the phosphorylation of a PKA substrate than does extracellular PKA derived from persons afflicted with carcinoma. Under certain other reaction conditions, extracellular PKA derived from persons unafflicted with carcinoma has less activity for the phosphorylation of a PKA substrate than does extracellular PKA derived from persons afflicted with carcinoma. Under yet other reaction conditions, extracellular PKA derived from persons unafflicted with carcinoma and extracellular PKA derived from persons afflicted with carcinoma have approximately equivalent activities for the phosphorylation of a PKA substrate.

Depending upon redox conditions apparent PKA activity in serum of cancer patients may be higher, lower, or the same as that of apparently healthy controls. Thus, apparent PKA activity may be used as an indicator of the presence of cancer, provided the redox conditions of the assay are known and/or controlled. Redox conditions are demonstrated herein that achieve each of the above relationships between apparent extracellular PKA activity in cancer patients and normal subjects, and preferred conditions are demonstrated for the use of this assay for detecting cancer.

In accordance with one aspect of the present invention, therefore, the results of an assay for an individual subject under a set of reaction conditions may be compared to a reference value for that set of reaction conditions to characterize carcinoma in that subject. For example, if the assay for the individual subject is carried out under reaction conditions at which the activity of extracellular PKA derived from subjects afflicted with carcinoma for the phosphorylation of a PKA substrate is significantly greater than the activity of extracellular PKA derived from subjects unafflicted with carcinoma for the phosphorylation of a PKA substrate (*i*.*e*., normal subjects), and the activity of the individual subject's PKA for the phosphorylation of PKA substrate is significantly greater than the activity that is characteristic of normal subjects, a diagnosis, prognosis, etc., may be determined. Alternatively, if the assay for the individual subject is carried out under reaction conditions at which the activity of extracellular PKA derived from subjects afflicted with carcinoma for the phosphorylation of a PKA substrate is significantly less than the activity of extracellular PKA derived from normal subjects for the phosphorylation of a PKA substrate, and the activity of the individual subject's PKA for the phosphorylation of PKA substrate is significantly less than the activity that is characteristic of subjects unafflicted with carcinoma, a diagnosis, prognosis, etc., may be determined.

The relative activities of extracellular PKA derived from subjects afflicted with carcinoma and normal individuals for the phosphorylation of a PKA substrate depends, at least in part, upon the oxidation state of the PKA in the assay. In general, the activity of extracellular PKA from normal individuals appears to be significantly influenced by the redox environment in which it is found. Extracellular PKA from normal individuals appears to have lower activity when the redox environment is oxidizing and higher activity when the environment is highly reducing. Consequently, the apparent activity of extracellular PKA in a fluid sample derived from normal individuals can be increased by treating the sample with a reducing agent to form a mixture that has an ORP value that is more reducing, or decreased by treating the sample with an oxidizing agent. In contrast, the activity of extracellular PKA derived from individuals afflicted with a carcinoma is relatively insensitive to oxidation state. That is, the activity is relatively constant irrespective of whether it is treated with an oxidizing agent or a reducing agent.

In one embodiment, apparent PKA activity is assayed after the sample is exposed to moderately reducing conditions, *i*.*e*., the range of conditions at which the apparent PKA activity in samples derived from cancer patients is greater than the apparent PKA activity that is characteristic of normal patients. Moderately reducing conditions may be established, for example, by forming a mixture comprising the sample and a reducing agent wherein the mixture has an ORP value in the range of about -110 mV to about -20 mV. For example, in one embodiment, the mixture containing the sample has an ORP value in the range of about -100 mV to about -90 mV. By way of further example, in one embodiment, the mixture containing the sample has an ORP value in the range of about -20 mV to about -30 mV.

In another embodiment, apparent PKA activity is assayed after the sample is exposed to oxidizing conditions, or mildly or highly reducing conditions, i.e., the range of conditions at which the apparent PKA activity in samples derived from cancer patients is less than the apparent PKA activity that is characteristic of normal patients. Highly reducing conditions may be established, for example, by forming a mixture comprising the sample and a reducing agent wherein the mixture has an ORP value that is less than about -110 mV (that is, conditions that are more reducing than about -110 mV). For example, in one embodiment, the mixture containing the sample has an ORP value of less than about -120 mV. By way of further example, in one embodiment, the mixture containing the sample has an ORP value of less than about -145 mV. Alternatively, mildly reducing or oxidizing conditions may be established, for example, by forming a mixture comprising the sample and an oxidizing agent or a reducing agent wherein the mixture has an ORP value of greater than -20 mV (that is, conditions that are more oxidizing than -20 mV). For example, in one embodiment, the mixture containing the sample has an ORP value of at least about -15 mV. By way of further example, in one embodiment, the mixture containing the sample has an ORP value of at least about 1 mV). By way of further example, in one embodiment, the mixture containing the sample has an ORP value of at least about 60 mV). By way of further example, in one embodiment, the mixture containing the sample has an ORP value of at least about 128 mV.

The sample may be incubated in a mixture having a desired, or at least known redox environment, for a period of time, before the assay is initiated. In certain embodiments, for example, the incubation time will be at least about 1 minute before the PKA activity assay is initiated. Typically, however, greater incubation times will be employed. For example, in one embodiment, the incubation time will be at least about 5 minutes. By way of further example, in some embodiments, the incubation time will be at least about 10 minutes. By way of further example, in some embodiments, the incubation time will be at least 30 minutes. By way of further example, in some embodiments, the incubation time will be at least about 1 hour. In such embodiments, the incubation temperature may be in the range of 20 to 37 °C, with about 25 °C being preferred in certain embodiments. This may be accomplished, for example, in an incubation mixture formed prior to the combination of the sample with the PKA substrate.

Although presently less preferred, in certain embodiments the sample is not incubated with an oxidizing agent or a reducing agent for a period of time before the PKA activity assay is initiated. Rather, a reaction mixture for determining PKA activity is prepared directly from the sample by combining the sample with a PKA peptide substrate, a phosphorylation agent, and optionally a reducing agent or oxidizing agent, the prepared mixture is incubated, and phosphorylated substrate formed in the incubated mixture is detected. In this situation it is generally preferred that the assay be carried out under reaction conditions at which a ratio of the activities for a statistically significant population of normal subjects to a statistically significant population of subjects afflicted with carcinoma be at least 0.05:1 and less than 0.8:1. In certain embodiments, it is preferred that the ratio of the activities for a statistically significant population of normal subjects to a statistically significant population of subjects afflicted with carcinoma be at least 0.075:1 and less than 0.6:1. In certain embodiments, it is preferred that the ratio of the activities for a statistically significant population of normal subjects to a statistically significant population of subjects afflicted with carcinoma be at least 0.1 and less than 0.4:1.

Because relative activities of apparent extracellular PKA from normal subjects and those afflicted with carcinoma depend upon reaction conditions, it is generally preferred that the reaction conditions for an assay be those at which the activities are significantly different. That is, it is generally preferred that the assay be carried out under reaction conditions at which a ratio of the activities for a statistically significant population of normal subjects to a statistically significant population of subjects afflicted with carcinoma be at least 1.2:1 or less than 0.8:1. In certain embodiments, it is preferred that the ratio of the activities for a statistically significant population of normal subjects to a statistically significant population of subjects afflicted with carcinoma be at least 2:1 or less than 0.5:1. In certain embodiments, it is preferred that the ratio of the activities for a statistically significant population of normal subjects to a statistically significant population of subjects afflicted with carcinoma be at least 3:1 or less than 0.2:1.

PKA has two exposed cysteines Cys¹⁹⁹ and Cys³⁴³. It has been determined that sulfhydryl modification of Cys³⁴³ has minimal impact on enzyme activity. However, sulfhydryl modification of Cys¹⁹⁹ predisposes the enzyme to dephosphorylation and inactivation [1]. Humphries et al demonstrated that apparent PKA activity in cell lysates is regulated by an interplay between oxidation of PKA and oxidation of phosphatases. [2] Because these enzymes differentially respond to oxidation, they react differentially to the redox state of the sample, and the overall apparent activity of PKA varies in a complex manner in response to the redox state of a sample.

We have demonstrated in serum that this complex interplay of PKA and phosphatase activities based on redox state also exists, and that a third regulatory mechanism of PKA activity may also be observed in blood. When the nonspecific phosphatase inhibitor NaF is added to a reaction mixture comprising serum derived from a normal subject, the apparent PKA activity is decreased by approximately 50%. In cell lysates when the nonspecific phosphatase inhibitor NaF is added to a PKA reaction mixture the apparent PKA activity increases if active phosphatases were present or remains the same if inactive phosphatases were present in the sample. Finding neither of these results, but rather a reduction in PKA activity in normal serum samples implies that a phosphatase-sensitive regulatory mechanism exists in serum from normal subjects that reduces the activity of PKA. A corresponding reduction in PKA activity in response to phosphatase inhibition, however, has not been observed, to-date, in serum from cancer patients.

When reaction conditions are selected that provide a significant difference in PKA activities for normal subjects as compared to those afflicted with a carcinoma, the results of the assay may be used to characterize a carcinoma in a subject. That is, the assay may be used to detect or diagnose cancer. In certain embodiments, it may also be used for the determination of a prognosis, determination of drug efficacy, monitoring the status of said subject's response or resistance to a treatment or selection of a treatment for said carcinoma.

In general, assays for determination of the activity of extracellular PKA may be carried out by preparing a reaction mixture comprising the sample, a phosphorylation agent, a PKA substrate, and a reagent or system for detecting phosphorylated substrate.

In general, the fluid sample may be derived from any bodily fluid of a subject or subjects. In one embodiment, the sample is previously unfrozen. Exemplary bodily fluids include peripheral blood, sera, plasma, ascites, urine, cerebrospinal fluid (CSF), sputum, saliva, bone marrow, synovial fluid, aqueous humor, amniotic fluid, cerumen, breast milk, broncheoalveolar lavage fluid, semen (including prostatic fluid), Cowper's fluid or pre-ejaculatory fluid, female ejaculate, sweat, fecal matter, hair, tears, cyst fluid, pleural and peritoneal fluid, pericardial fluid, lymph, chyme, chyle, bile, interstitial fluid, menses, pus, sebum, vomit, vaginal secretions, mucosal secretion, stool water, pancreatic juice, lavage fluids from sinus cavities, bronchopulmonary aspirates or other lavage fluids. Additional exemplary bodily fluids include the blastocyl cavity, umbilical cord blood, or maternal circulation which may be of fetal or maternal origin. In one exemplary embodiment, the fluid sample is derived from a bodily fluid selected from among whole blood, sputum, serum, plasma, urine, cerebrospinal fluid, nipple aspirate, saliva, fine needle aspirate, and combinations thereof. In another exemplary embodiment, the fluid sample is derived from a bodily fluid selected from among whole blood, serum, plasma, urine, nipple aspirate, saliva, and combinations thereof. In one preferred embodiment, the fluid sample is derived from blood plasma or serum.

In one embodiment, the sample is treated with a reducing agent or an oxidizing agent. This treatment step may be carried out before the sample is combined with the other components of the reaction mixture, or along with the other components of the reaction mixture.

Reducing agents such as 2-mercaptoethanol, Syringaldazine, sodium hydrosulfite, dithiothreitol, dithioerythreitol,and tris(2-carboxyethyl) phosphine hydrochloride (TCEP) may be used as reducing agents to affect the redox state of the PKA reaction. The reducing agents may preferably be used be at concentrations between 50 uM and 100 mM. The reducing agents may be mixed with the sample prior to addition of the sample to the assay, or the reducing agents may be incorporated into the assay mixture. The sample, separately or in the reaction mixture, may be incubated with the reducing agents preferably between 1 minute and 60 minutes.

Oxidizing agents such as diamide, or hydrogen peroxide may be used as oxidizing agents to affect the redox state of the PKA reaction. The oxidizing agents may preferably be used be at concentrations between 5 uM and 100 mM. The reducing agents may be mixed with the sample prior to addition of the sample to the assay, or the reducing agents may be incorporated into the assay mixture. The sample, separately or in the reaction mixture, may be incubated with the reducing agents preferably between 1 minute and 60 minutes

The phosphorylation agent will typically be ATP although other phosphorylation agents may be employed in certain embodiments.

In general, the PKA substrate may be any peptide substrate for PKA. Exemplary PKA substrates include histone Ila. In a preferred embodiment, the PKA substrate is Kemptide (Leu-Arg-Arg-Ala-Ser-Leu-Gly).

A specific inhibitor of PKA may be useful to discriminate PKA activity from other related kinase activity. One PKA-specific inhibitor which may be used for this purpose is PKI peptide (Ile-Ala-Ala-Gly-Arg-Thr-Gly-Arg-Arg-Gin-Ala-Ile-His-Asp-Ile-Leu-Val-Ala-Ala-OH). Related peptides and shorter peptides derived from the PKI sequence also may be used as PKA-specific inhibitors.

The phosphorylated substrate may be detected using a variety of systems. In general, a probe having affinity for the phosphorylated substrate is conjugated to a "functional group" which is directly or indirectly detectable. The probe may be, for example, an antiphosphoserine antibody. The functional group may be a moiety which is measurable by direct or indirect means (e.g., a radiolabel, a photoactivatable molecule, a chromophore, a fluorophore or a luminophore), or spectroscopic colorimetric labels such as colloidal gold or colored glass or plastic (*e*.*g*. polystyrene, polypropylene, latex, etc.) beads. By way of further example, the functional group may be a moiety that is indirectly detectable such as an enzyme (*e*.*g*., horse radish peroxidase, alkaline phosphatase etc.), biotin, or a hapten such as digoxigenin. In one exemplary embodiment, an antibody probe is conjugated to a functional group such as a radiolabel, fluorophore, chromophore, chemiluminescent moiety, or enzyme, to facilitate detection. In another embodiment, the probe is conjugated to one member of an affinity pair, *e*.*g*., biotin, and a detectable label is conjugated to the second member of the affinity pair, *e*.*g*., avidin or streptavidin.

Exemplary radiolabels include ³H, ¹²⁵I, ³⁵S, ¹⁴C, ³²P, and ³³P.

Exemplary chromophore/luminophores include any organic or inorganic dyes, fluorophores, phosphophores, light absorbing nanoparticles (*e.g.,* Au, Ag, Pt, Pd), combinations thereof, or the metalated complexes thereof.

Exemplary organic dyes are selected from the group consisting of coumarins, pyrene, cyanines, benzenes, N-methylcarbazole, erythrosin B, N-acetyl-L-tryptophanamide, 2,5-diphenyloxazole, rubrene, and N-(3-sulfopropyl)acridinium. Specific examples of preferred coumarins include 7-aminocoumarin, 7-dialkylamino coumarin, and coumarin 153. Exemplary benzenes include 1,4-bis(5-phenyloxazol-2-yl)benzene and 1,4-diphenylbenzene. Exemplary cyanines include oxacyanines, thiacyanines, indocyanins, merocyanines, and carbocyanines. Other exemplary cyanines include ECL Plus, ECF, C3-Oxacyanine, C3-Thiacyanine Dye (EtOH), C3-Thiacyanine Dye (PrOH), C5-Indocyanine, C5-Oxacyanine, C5-Thiacyanine, C7-Indocyanine, C7-Oxacyanine, CypHer5, Dye-33, Cy7, Cy5, Cy5.5, Cy3Cy5 ET, Cy3B, Cy3, Cy3.5, Cy2, CBQCA, NIR1, NIR2, NIR3, NIR4, NIR820, SNIR1, SNIR2, SNIR4, Merocyanine 540, Pinacyanol-Iodide, 1,1-Diethyl-4,4-carbocyanine iodide, Stains All, Dye-1041, or Dye-304.

Exemplary inorganic dyes include metalated and non-metalated porphyrins, phthalocyanines, chlorins (*e.g.,* chlorophyll A and B), and metalated chromophores. Exemplary porphyrins include porphyrins selected from the group consisting of tetra carboxy-phenyl-porphyrin (TCPP) and Zn-TCPP. Exemplary metalated chromophores include ruthenium polypyridyl complexes, osmium polypyridyl complexes, rhodium polypyridyl complexes, 3-(1-methylbenzoimidazol-2-yl)-7-(diethylamino)-coumarin complexes of iridium(III), and 3-(benzothiazol-2-yl)-7-(diethylamino)-coumarin complexes with iridium(III).

Exemplary fluorophores and phosphophores include phosphorescent dyes, fluoresceines, rhodamines (*e*.*g*., rhodamine B, rhodamine 6G), and anthracenes (*e*.*g*., 9-cyanoanthracene, 9,10-diphenylanthracene, 1-Chloro-9,10-bis(phenylethynyl)anthracene).

As previously noted, any one or more of a range of other general cancer assays may be employed in addition to, or as an alternative to, the extracellular PKA assay described above. For example, the Onco-Sure (AMDL DR-70) test is used to detect fibrin and fibrin degradation products in serum. The Onco-Sure test has been used to detect breast, lung, colon, liver, ovarian, pancreatic, stomach, rectal, cervical, thyroid, esophageal, and gastric cancers. The sensitivity of the test for general cancer detection is 84-91 % with the sensitivity for detection of some specific cancers, such as breast cancer, as low as 65% [25-28]. Another exemplary general cancer assay involves the use of PCNA (proliferating-cell nuclear antigen), which has been identified in cancer at many organ sites and may be used a general cancer biomarker. PCNA expression detected by immunostaining was particularly evident in later stage disease with expression detected in up to 85% of late stage cancers [29-41]. Loveday and Greenman proposed a panel of tumor-associated biomarkers as a general test of cancer [42]. The presence of hTR of hTERT extracellular RNA was detected in the 72% (13 0f 18) pancreatic cancer patients [43] and was implied to be a general cancer biomarker.

As noted above, nucleic acids can been analyzed in many ways to detect cancer. Methylated DNA is just one of the epigenetic methods used to regulate normal gene expression. In carcinogenesis, abnormal patterns of DNA methylation occur that can be indicative of the cancerous state [2, 6, 8-9, 44-46]. The expression of small miRNAs which destabilize messenger mRNAs can be altered and indicative of cancer. This latter biomarker group also can be used determine the subtype of a particular organ cancer or to determine patient prognosis [3, 47-48].

Other indicators and biomarkers that may be used in accordance with the general screening methods described herein include Sga-1m, Tadg-15, Ephrin type-A receptor 10 protein, hTR and hTERT RNA, a serum inhibitor of carbonic anyhdrase, and others [49-63].

### ORGAN-SPECIFIC CANCER BIOMARKERS

A range of cancer indicators have been identified in biological fluids. Such markers and indicators may be comprised of, or derived from, proteins, peptides, antibodies, autoantibodies, lipids, sugars, nucleic acids, DNA, RNA, mRNA, methylated DNA, and circulating or cultured tumor cells. In one particular embodiment, the organ-specific cancer markers are identifiable in whole blood, plasma, serum, or urine. Although protein (or other) biomarkers are often used to determine disease stage or monitor the disease or determine prognosis, these biomarkers can be used, for instance, in combination with the extracellular PKA assay described above to determine the site of cancer once a patient or subject is found to be positive for cancer. Using the organ-specific cancer diagnostics in this manner is particularly effective as it can decrease the number of conventional organ-specific tests that would need to be done to detect specific cancers even before a cancer diagnosis could be confirmed, and would thereby lower the cost per case of organ-specific cancer detected.

The tumor marker(s) employed in the organ-specific screening assays will typically vary depending on the organ of interest. It will be understood, however, that there may be some overlap, as some tumor markers may be involved in more than one cancer type or origin.

In general, any of a number of tumor-specific markers associated with a range of organs and tumor types can be employed in the organ-specific screening methods described herein. Representative markers include those found in Table 1:

**Table 1: Organ site cancer biomarkers**

| **Organ/Site of Cancer** | **Marker** |
|---|---|
| Lung | CYFRA21-1, TPA-M, TPS, CEA, SCC-Ag, XAGE-1b, HLA Class 1, TA-MUC1, KRAS, hENT1, kinin B1 receptor, kinin B2 receptor, TSC403, HTI56, DC-LAMP, p53, c-erbB2, PHT-RP, vasopressin, gastrin releasing peptide, Annexins I and II, Hu, KOC, chromogranin A, EGFR |
| Colon/Colorectal | GPA33, CEA (e.g., CEACAM5), ENFB1, CCSA-2, CCSA-3, CCSA-4, ADAM10, CD44, NG2, ephrin B1, plakoglobin, galectin 4, RACK1, tetraspanin-8, FASL, A33, CEA, EGFR, dipeptidase 1, PTEN, Na(+)-dependent glucose transporter, UDP-glucuronosyltransferase 1A, CEA, CA19-9, K-ras, SMAD7, p53, c-erbB2, ras, APC, pro-gastrin, gastrin G17, gastrin G34, PTH-RP, CA242, TIMP-1, DCC, DPD, TS, CK-19, CK-20, REG-4, TIAM1 |
| Prostate | PSA, TMPRSS2, FASLG, TNFSF10, PSMA, NGEP, II-7RI, CSCR4, CysLT1R, TRPM8, Kv1.3, TRPV6, TRPM8, PSGR, MISIIR, galectin-3, PCA-3, TMPRSS2:ERG, NF-kappa-B, CEA, p53, c-erbB2, BRCA1, kallikrein, PTH-RP, PAP |
| Brain | PRMT8, BDNF, EGFR, DPPX, Elk, Densin-180, BAI2, BAI3 |
| Blood | CD44, CD58, CD31, CD11a, CD49d, GARP, BTS, Raftlin |
| Testicles | human chorionic gonadotropin (HCG), lactate dehydrogenase (LDH), alpha fetoprotein (AFP), beta-hCG |
| Breast | BRCA1, BRCA2, HER2/neu, CA 15-3, CEA, CA 27.29, TGF-beta-1, cyclin E, |
| | MUC1, p53, c-erbB2, c-myc, PSA, CYFRA21-1, PTH-RP, EGFR |
| Skin/melanoma | DUSP1, TYRP1, SILV, MLANA, MCAM, CD63, Alix, hsp70, meosin, p120 catenin, PGRL, syntaxin binding protein 1 &2, caveolin, TA-90, S-100 |
| Liver | HBxAg, HBsAg, NLT, alpha fetoprotein (AFP), GP73, p53, c-erbB2, p62 |
| Cervix | MCT-1, MCT-2, MCT-4, SCC, CA 125, p53, c-erbB2, HPV (and sub-types thereof), beta-hCG, urinary gonadotropic fragment, alpha fetoprotein (AFP), inhibin, estradiol, CEA, MIS, topoisomerase II, CA 19-9, CA 27-29, hTERT, ferritin |
| Ovaries | CA 125, CA 72-4, CEA, LASA-P, human chorionic gonadotropin (HCG), HE4, MUC1, p53, c-erbB2 c-myc, BRCA1, PTH-RP, beta-hCG, urinary gonadotropic fragment, alpha fetoprotein (AFP), inhibin, estradiol, CEA, SCC, MIS, topoisomerase II, CA 19-9, CA 27-29, hTERT, ferritin |
| Endometrium | Alpha V Beta 6 integrin, CA 125, beta-hCG, urinary gonadotropic fragment, alpha fetoprotein (AFP), inhibin, estradiol, CEA, SCC, MIS, topoisomerase II, CA 19-9, CA 27-29, hTERT, ferritin |
| Bladder | bladder tumor antigen (BTA), NMP22, CEA, CA 125, CA 19-9, TPA, MUC1, p53, c-erbB2, c-myc |
| Leukemia | Bcr-abl, Beta-2-microglobulin, calcitonin, CD52, ferritin, WT1 |
| Pancreas | CA 19-9, CEA, PAM4, p53, c-erbB2, CA 72-4, EGFR, DPC4, CDKN2 |
| Kidney | AQP1, ADFP, TSC1, TSC2, VHL |
| Gastrointestinal | CEA, gastrin G17, gastrin G34, pro-gastrin, glucagon, CA 19-9, CA 72-4, p53 |

It will be understood that family members, fragments, antibodies, antigens, recombinant versions, mutated versions, binding agents, and cell surface agents of the above markers may additionally or alternatively be employed. Combinations of the above markers may also be employed.

Certain preferred markers for the second (*i*.*e*., organ-specific) assay include the epidermal growth factor receptor-related protein c-erbB2 (Dsouza, B. et al. (1993) Oncogene. 8: 1797-1806), the glycoprotein MUC1 (Batra, S. K. et al. (1992) Int. J. Pancreatology. 12: 271-283) and the signal transduction/cell cycle regulatory proteins Myc (Blackwood, E. M. et al. (1994) Molecular Biology of the Cell 5: 597-609), p53 (Matlashewski, G. et al. (1984) EMBO J. 3: 3257-3262; Wolf, D. et al. (1985) Mol. Cell. Biol. 5: 1887-1893) and ras (or Ras) (Capella, G. et al. (1991) Environ Health Perspectives. 93: 125-131), including the viral oncogenic forms of ras which can be used as antigens to detect anti-ras autoantibodies, and also BRCA1 (Scully, R. et al. (1997) PNAS 94: 5605-10), BRCA2 (Sharan, S. K. et al. (1997) Nature. 386: 804-810), APC (Su, L. K. et al. (1993) Cancer Res. 53: 2728-2731; Munemitsu, S. et al. (1995) PNAS 92: 3046-50), CA125 (Nouwen, E. J. et al. (1990) Differentiation. 45: 192-8) and PSA (Rosenberg, R. S. et al. (1998) Biochem Biophys Res Commun. 248: 935-939). Additional markers which might also be used include CEA gene family members, PTH-RP, CYFRA21-1, kallikrein, pro-gastrin, gastrin G17, gastrin G34, CA19-9, CA72-4, vasopressin, gastrin releasing peptide, SCC, TK, αFP, p62, annexins I and II, Hv and KOC or antigens of HPV, preferably sub-types associated with cancer risk. As noted above, the assays can be performed using tumor marker antigens which are forms of these proteins isolated from human bodily fluids or from cultured cells or antigenic fragments thereof or full length or truncated recombinant proteins or antigenic fragments thereof.

In another embodiment, one of the biomarkers is an AMACR autoantibody, or a MUC autoantibody. By way of further example, one of the biomarkers may be organ-specific tumor-related methylated DNA, organ-specific tumor-related miRNA, or an organ-specific tumor-related circulating nucleic acid biomarker.

In one preferred embodiment, the organ-specific cancer tests selected for use in the methods described herein have a 70% or better sensitivity for detection of specific cancers. For example, the biomarkers listed in Table 2 may be employed in one or more organ-specific cancer screens [64-85]:

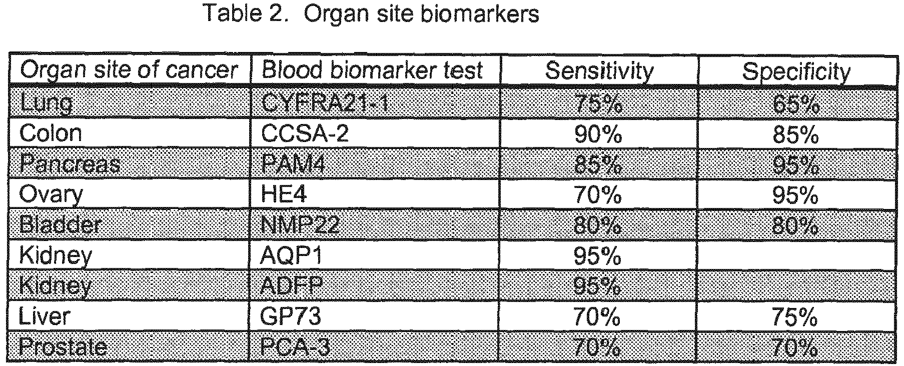

In other, generally less preferred embodiments, an organ-specific biomarker with a sensitivity of <70% may be used. One such example is the PSA (prostate-specific antigen) blood test used as a screening test for prostate cancer.

Multimarker patterns of biomarkers also have been used to detect specific cancers. These multivariate tests include tests for mRNA expression, protein biomarkers, and autoantibodies [91-105]. Variation in the expression of mRNA and mRNA expression patterns also have been used to detect specific cancers [12, 106-110].

The expression or expression level of the tumor- or organ-specific markers described herein can be determined or detected by any detection means known in the art, including, but not limited to, subjecting the sample to an analysis selected from the group consisting of immunological assays (such as ELISA, radioimmunoassays, and the like), fluorescence co-localization analysis, fluorescence in situ hybridization, polymerase chain reaction (PCR)-based methods (such as real time PCR, quantitative RT-PCR analysis, and the like), ribonuclease protection assays, S1 nuclease assays, Northern blot analysis, combinations thereof, and the like.

In general terms, where immunological assays are employed, such assays use an antigen which may be immobilized on a solid support. A biological sample (or portion thereof) to be tested is brought into contact with the antigen and if autoantibodies specific to the tumor marker protein are present in the sample they will immunologically react with the antigen to form autoantibody-antigen complexes which may then be detected or quantitatively measured. Detection of autoantibody-antigen complexes is preferably carried out using a secondary anti-human immunoglobulin antibody, typically anti-IgG or anti-IgM, which recognize general features common to all human IgGs or IgMs, respectively. The secondary antibody is usually conjugated to an enzyme such as, for example, horseradish peroxidase (HRP) so that detection of autoantibody/antigen/secondary antibody complexes is achieved by the addition of an enzyme substrate and subsequent colorimetric, chemiluminescent or fluorescent detection of the enzymatic reaction products.

Depending on the particular cancer type, patient sample, and/or tumor marker being analyzed, the assays described herein can be directed to a single tumor marker or a group of two or more (e.g., 5, 10, 15, 20, and so on) tumor markers. In certain embodiments, the specific tumor assay portion of the analysis may be conducted as part of a panel assay. A panel assay of the present invention uses a panel of tumor marker-related antigens. The panel may be tailored, for example, to detect a particular cancer or a range of different cancers, or a cancer at a particular stage of development. The tumor marker antigens may be wild type or mutant tumor marker proteins isolated from samples of biological fluid from normal individuals or from cancer patients or from cell lines expressing the tumor marker protein, or they may be full length recombinant tumor marker proteins, viral oncogenic forms of tumor marker proteins or antigenic fragments (*e*.*g*., a fragment capable of eliciting an immune response) of any of the aforementioned proteins. The panel assays may be performed in a multi-well format in which each one of the two or more antigens is placed in separate wells of multi-well assay plates or, alternatively, in a single-pot format in which the entire panel of antigens is placed in a single container. The panel assays may be performed in a qualitative format in which the objective is simply detection of the presence or absence of autoantibodies or in a quantitative format which provides a quantitative measurement of the amount of autoantibodies present in a sample.

### ADDITIONAL CANCER INDICATORS

Other cancer indicators and screening methods may additionally be employed in prior to or subsequent to the assay methods described herein. These include, for example, ultrasounds, X-rays, laparoscopy, paracentesis, mammograms, biopsies, colonoscopies, body scans (e.g., MRI, infra-red, CT scan, etc.), and the like. By way of example, once a patient receives a positive (or even a negative) test result based upon the general and specific assays described herein, additional screening modalities may be performed.

### EXAMPLES

The following non-limiting examples are provided to further illustrate the present invention. It should be appreciated by those of skill in the art that the techniques disclosed in the examples that follow represent approaches the inventors have found function well in the practice of the invention, and thus can be considered to constitute examples of modes for its practice. However, those of skill in the art should, in light of the present disclosure, appreciate that many changes can be made in the specific embodiments that are disclosed and still obtain a like or similar result without departing from the spirit and scope of the invention.

### EXAMPLE 1

Serum samples from patients cancer and individuals apparently without cancer were obtained from ProMedDx, LLC and from ProteoGenex. In one embodiment blood samples from prostate cancer patients and normal controls presumably without cancer were assayed for apparent PKA activity. In this assay extracellular PKA in samples was mixed with a defined peptide used as a substrate. The substrate peptide was bound to the wells of the microtiter assay plate. Phosphorylation of the peptide was detected using biotinylated phosphoserine antibody, which was in turn was detected in an ELISA format using peroxidase-conjugated to streptavidin. Detection of the bound peroxidase was established using a color-producing peroxidase substrate included in the assay kit. Bovine PKA catalytic unit was used at varying concentrations to develop a standard activity curve. The detail of the assay protocol is described below.
1. Reference: Kit Instructions
2. Materials
   a. MESACUP Protein Kinase Assay Kit Components (MBL Code No. 5230)
   b. ATP: 10 mM in water
      i. Dissolve 60 mg ATP (Sigma Prod. No. A2383) in 1.0 ml water
      ii. Determine the absorbance of a 1/1000 dilution in PBS at 259 nm
      iii. Store at -20°C
      iv. Immediately before use dilute to 10 mM based on the absorbance and the molar extinction coefficient (E₂₅₉, pH 7 = 15,400)
   c. PKI inhibitor: 0.5 mM in water (Santa Cruz Prod. No. sc-201160)
      i. Dissolve 1 mg in 1.0 ml water
      ii. Store at -20°C
   d. PKA diluent: 25 mM KH₂PO₄, 5 mM EDTA, 150 mM NaCl, 50% (w/v) glycerol, 1 mg/ml BSA, and various concentrations of reductant or oxidant (2-mercaptoethanol, dithiothreitol, dithioerythritol, or diamide) as indicated directly in the data figures by concentration or by oxidation-reduction potential, pH 6.5
   e. PKA catalytic subunit standard:
      i. Dissolve bovine PKA (Sigma Prod. No. P2645) in cold PKA diluent to a final concentration of 1 µg/ml
      ii. Store at -20°C
   f. Peroxidase substrate solution (Sigma Prod. No. T8665)
3. Procedure
   g. Prepare samples
      i. Thaw serum samples
      ii. Centrifuge 5 minutes at 16,000 x g
      iii. Collect the clear supernatant
      iv. Mix 0.0108 ml supernatant with 0.0012 ml diluent in a dilution plate, two wells per sample
      v. Incubate one hour at room temperature
   h. Prepare calibration curve
      i. Prepare serial 1/2 dilutions of PKA 20 - 0.4 ng/ml in PKA diluent
      ii. Dispense 0.012 ml per well of a dilution plate
   i. Prepare reaction buffer to final concentrations of:
      i. 25 mM tris-HCl, pH 7.0
      ii. 3 mM MgCl₂
      iii. 1 mM ATP
      iv. 0 or 5 uM PKI
   j. Add 0.108 ml reaction buffer (with or without PKI) to each sample or calibrator well of the dilution plate
   k. Pre-incubate five minutes at 25°C
   l. Transfer 0.100 ml per well to assay plate
   m. Incubate 20 minutes at 25°C with shaking at 750 rpm
   n. Add 0.100 ml kit stop solution per well
   o. Wash three times with kit wash buffer
   p. Add 0.100 ml kit biotinylated anti-phosphoserine per well
   q. Incubate 60 minutes at 25°C with shaking at 750 rpm
   r. Wash three times with kit wash buffer
   s. Add 0.100 ml kit peroxidase-conjugated streptavidin per well
   t. Incubate 60 minutes at 25°C with shaking at 750 rpm
   u. Wash three times with kit wash buffer
   v. Add 0.100 ml peroxidase substrate solution per well
   w. Incubate 3 minutes at 25°C with shaking at 750 rpm
   x. Add 0.100 ml kit stop solution per well
   y. Shake briefly until well mixed
   z. Read absorbance at 450 nm
4. Calculation of results
   aa. Plot absorbance versus concentration of the calibration curve
   bb. Perform a least squares linear regression on the data to determine the slope and intercept
   cc. Calculate kinase concentration in the samples
      i. Kinase (ng/ml) = (sample absorbance - intercept)/slope
   dd. Calculate net PKA in the samples
      i. Net PKA (ng/ml) = Kinase (0 uM PKI) - Kinase (0.5 uM PKI).

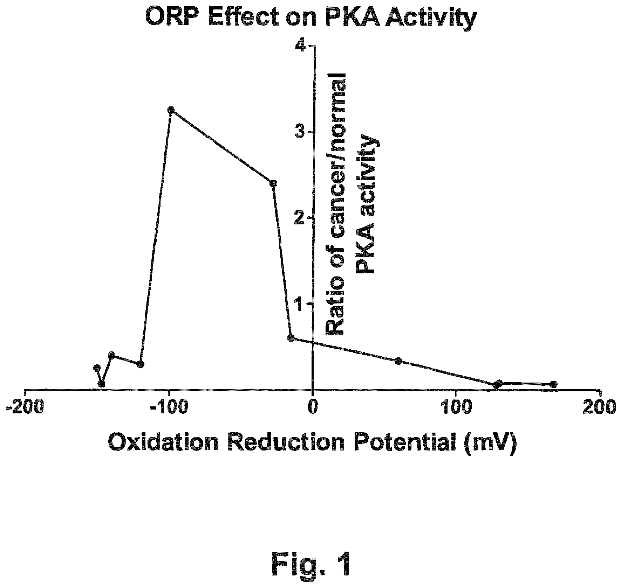

The oxidation-reduction potential (ORP) of the sample preparation buffers was measured using a platinum redox probe. ORPs are expressed in mV. The apparent PKA activity for samples from cancer patients relative to normal samples were plotted for various ORP value solutions. As shown in Figure 1, under oxidizing conditions, mild reducing conditions or highly reducing conditions the apparent PKA activity in serum samples from prostate cancer patients was lower than that from samples from individuals apparently without cancer. Under moderate reducing conditions, the apparent PKA activity in serum samples from prostate cancer patients was higher than that from samples from individuals apparently without cancer.

Table 3 shows the relative apparent PKA activities in samples from prostate and colon cancer patients relative to those from individuals apparently without cancer with various concentrations of oxidant or reductant used in the sample preparation buffer. The same pattern of PKA activity is observed with both the prostate and the colon cancer patient samples. The relative PKA activities of cancer patients is higher, lower, or the same as that of individuals apparently without cancer, depending upon the concentration of oxidant or reductant used in the sample preparation buffer.

**TABLE 3. ORP AND RELATIVE PKA ACTIVITIES**

| | Ratio of PKA Activity | | |
|---|---|---|---|
| | cancer/normal | | Eh |
| | Prostate | Colon | (mV) |
| no reductant | 0.07 | | +168 ^{.} |
| 2 mM diamide | 0.08 | | +130 |
| 100 uM diamide | 0.06 | 0.21 | +128 |
| | | | |
| 0.5 mM DTT | 3.25 | 1.22 | -100 |
| 5 mM DTT | 0.94 | 0.40 | -140 |
| 10 mM DTT | | 0.25 | -150 |
| | | | |
| 0.5 mM DTE | 2.40 | 1.13 | -28 |
| 5 mM DTE | 0.30 | 0.02 | -120 |
| | | | |
| 0.05 mM BME | 0.34 | | +60 |
| 0.5 mM BME | 0.60 | | -15 |
| 5 mM BME | 0.38 | | -85 |
| 50 mM BME | 0.07 | | -147 |

### EXAMPLE 2

PKA activities were determined using the procedure described in Example 1 with the exception that oxidant addition, reductant addition, or no addition was made to the reaction buffer. Samples were not preincubated in sample buffer, but were incubated for 5 minutes at 25 C in reaction buffer with shaking at 750 rpm prior to adding the reaction mixtures to the assay plate wells.

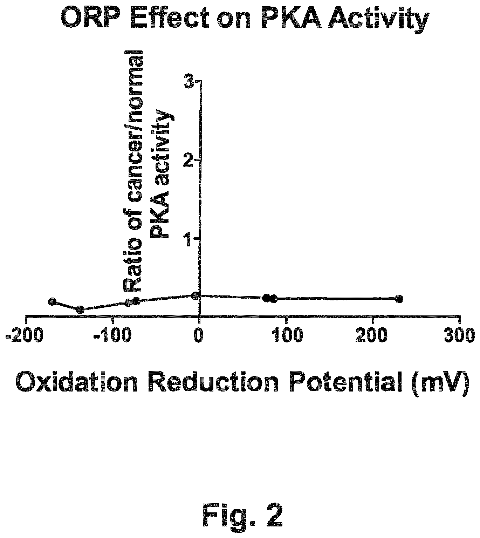

The oxidation-reduction potential (ORP) of the reaction buffers was measured using a platinum redox probe. ORPs are expressed in mV. The apparent PKA activity for samples from cancer patients relative to normal samples were plotted for various ORP value solutions. With a shorter treatment with oxidant or reductant (Figure 2) there is lower overall activity observed in the samples and the level of apparent PKA activity of the cancer patient samples relative to those from normals is consistently low (below 0.4:1)

### EXAMPLE 3

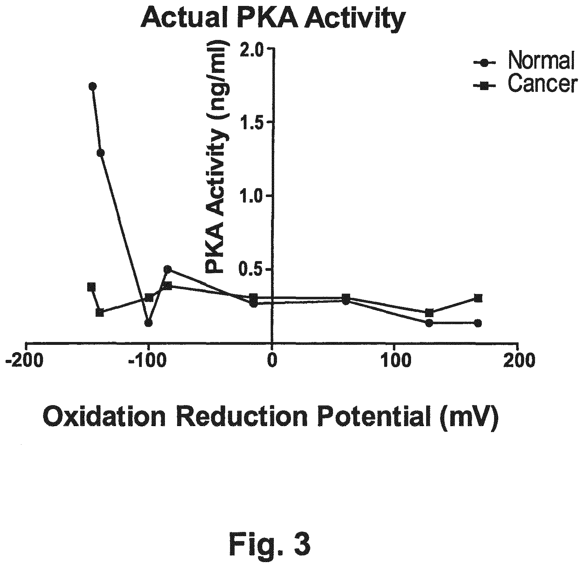

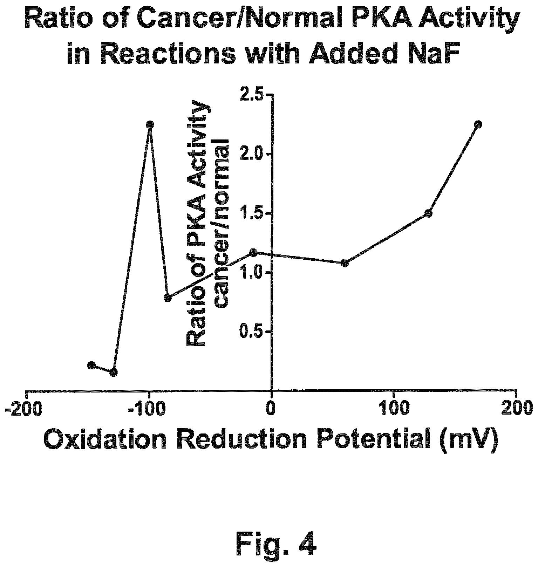

Samples were treated as in example 1. In one set of reaction mixes NaF was added at a concentration of 2 mM. NaF is a nonspecific inhibitor of phosphatases. A reaction run with NaF inhibitor provides a measure of actual PKA activity. Figure 3 shows that with phosphatase inhibition, the actual PKA activity in samples from cancer patients varied little with changes in redox conditions. In normal subjects with phosphatase inhibition however, actual PKA activity was reduced by about 50% overall and the PKA was still subject to regulation by redox conditions. This demonstrates the complex interplay of enzyme activities and redox conditions that control apparent PKA activity and that controls the relationship between apparent PKA activity levels in cancer patients and those individuals apparently without cancer. Fig. 4 shows the ratio of apparent PKA activity (cancer subjects/normal subjects) as a function of oxidation reduction potential in reactions containing NaF. The ratio of cancer/normal PKA activity varies dramatically depending upon redox conditions.

### EXAMPLE 4

In another embodiment of the invention serum samples from patients without cancer and patients with various types of cancer including lung cancer were obtained from ProMedDx and from ProteoGenex. The samples were tested for activated PKA activity (PKA diluent used: 25 mM KH₂PO₄, 50 mM EDTA, 150 mM NaCl, 50% (w/v) glycerol, 1 mg/ml BSA, and 50 mM 2-mercaptoethanol, pH 6.5; samples were incubated in PKA diluent for 30 min at room temperature prior to assay) and the extracellular PKA activity values for the samples are shown in Table 4. The same samples were assayed for CYFRA21-1 concentration according to the kit instructions of the supplier (Fujirebio, AB). CYFRA21-1 values for these samples also are shown in Table 4. Of the four samples from lung cancer (NSCLC) patients, all have low activated serum PKA levels (< 5 ng/ml). Samples from three of the patients also have elevated CYFRA21-1 values (> 2.8 ng/ml) indicating that they have lung cancer. The supplier indicates that approximately 75% of lung cancer patients will have elevated CYFRA21-1 concentrations, as was found, These data provide an example of how activated PKA activity and an organ-specific biomarker can be used to identify an individual with cancer and determine that the individual has specific type of cancer.

### EXAMPLE 5

In another embodiment of the invention serum samples from patients without cancer and patients with various types of cancer including lung cancer were obtained from ProMedDx and from ProteoGenex. The samples were tested for extracellular activated serum PKA using the same procedure described for the data in Table 4. The extracellular PKA activity values for the samples are shown in Table 5. The same samples were assayed for PSA concentration according to the kit instructions of the supplier (Calbiotech). PSA values for these samples also are shown in Table 5. The three samples from prostate cancer patients (samples 1-3) have low activated serum PKA levels (< 5 ng/ml) and also have elevated PSA values (> 4 ng/ml). This provides an example of how the measurement of activated PKA activity and PSA concentration can be used to identify an individual with cancer and determine that the individual has prostate cancer. Sample number 19 was provided as that of an apparently healthy male. However, this individual has a low activated serum PKA level of 4.7 ng/ml and an elevated PSA level. These data would indicate that this individual has a previously undetected case of prostate cancer. This provides a further example of how PKA activity and PSA levels can be used to identify an individual with prostate cancer

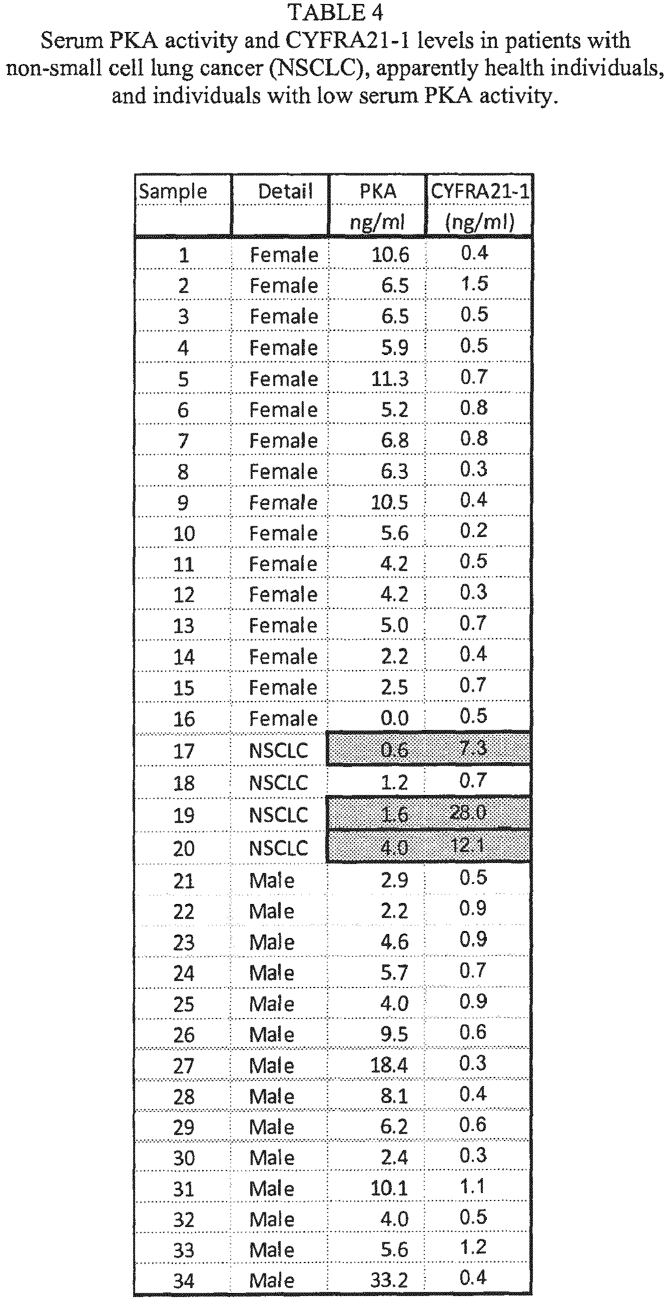

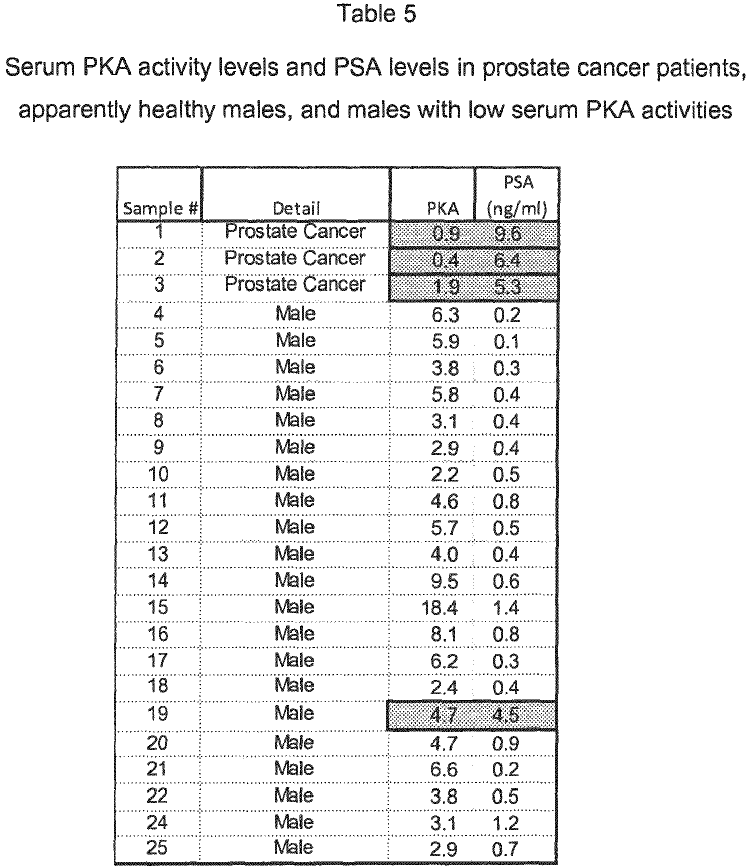

### REFERENCES

1. Zimmerman, A.L. and S. Wu, MicroRNAs, cancer and cancer stem cells. Cancer Lett, 2011. 300(1): p. 10-9.
2. Taberlay, P.C. and P.A. Jones, DNA methylation and cancer. Prog Drug Res, 2011. 67: p. 1-23.
3. Farazi, T.A., et al., miRNAs in human cancer. J Pathol, 2011. 223(2): p. 102-15.
4. Wang, D. and R.N. Dubois, Eicosanoids and cancer. Nat Rev Cancer, 2010. 10(3): p. 181-93.
5. Theocharis, A.D., et al., Proteoglycans in health and disease: novel roles for proteoglycans in malignancy and their pharmacological targeting. FEBS J, 2010. 277(19): p. 3904-23.
6. Taby, R. and J.P. Issa, Cancer epigenetics. CA Cancer J Clin, 2010. 60(6): p. 376-92.
7. Silvera, D., S.C. Formenti, and R.J. Schneider, Translational control in cancer. Nat Rev Cancer, 2010. 10(4): p. 254-66.
8. Shivapurkar, N. and A.F. Gazdar, DNA methylation based biomarkers in non-invasive cancer screening. Curr Mol Med, 2010. 10(2): p. 123-32.
9. Lechner, M., C. Boshoff, and S. Beck, Cancer epigenome. Adv Genet, 2010. 70: p. 247-76.
10. Kosaka, N., H. Iguchi, and T. Ochiya, Circulating microRNA in body fluid: a new potential biomarker for cancer diagnosis and prognosis. Cancer Sci, 2010. 101(10): p. 2087-92.
11. Khabar, K.S., Post-transcriptional control during chronic inflammation and cancer: a focus on AU-rich elements. Cell Mol Life Sci, 2010. 67(17): p. 2937-55.
12. Chen, C., et al., Microfluidic isolation and transcriptome analysis of serum microvesicles. Lab Chip, 2010. 10(4): p. 505-11.
13. Bozza, P.T. and J.P. Viola, Lipid droplets in inflammation and cancer. Prostaglandins Leukot Essent Fatty Acids, 2010. 82(4-6): p. 243-50.
14. Boffetta, P., Biomarkers in cancer epidemiology: an integrative approach. Carcinogenesis, 2010. 31(1): p. 121-6.
15. Bell, D.W., Our changing view of the genomic landscape of cancer. J Pathol, 2010. 220(2): p. 231-43.
16. Smith, B.K., et al., Trans-fatfy acids and cancer: a mini-review. Br J Nutr, 2009. 102(9): p. 1254-66.
17. Sequist, L.V., et al., The CTC-chip: an exciting new tool to detect circulating tumor cells in lung cancer patients. J Thorac Oncol, 2009. 4(3): p. 281-3.
18. Fernandis, A.Z. and M.R. Wenk, Lipid-based biomarkers for cancer. J Chromatogr B Analyt Technol Biomed Life Sci, 2009. 877(26): p. 2830-5.
19. Wang, Y., et al., Gene expression profiles and prognostic markers for primary breast cancer. Methods Mol Biol, 2007. 377: p. 131-8.
20. Masters, J.R., Clinical applications of expression profiling and proteomics in prostate cancer. Anticancer Res, 2007. 27(3A): p. 1273-6.
21. Pearce, L.R., D. Komander, and D.R. Alessi, The nuts and bolts of AGC protein kinases. Nat Rev Mol Cell Biol, 2010. 11(1): p. 9-22.
22. Walsh, D.A., et al., The inhibitor protein of the cAMP-dependent protein kinase, in Peptides and Protein Phosphorylation, B.E. Kemp, Editor. 1990, CRC Press, Inc.: Boca Raton, FL. p. 43-84.
23. Cho, Y.S., Y.N. Lee, and Y.S. Cho-Chung, Biochemical characterization of extracellular cAMP-dependent protein kinase as a tumor marker. Biochem Biophys Res Commun, 2000. 278(3): p. 679-84.
24. Cho, Y.S., et al., Extracellular protein kinase A as a cancer biomarker: its expression by tumor cells and reversal by a myristate-lacking Calpha and Rllbeta subunit overexpression. Proc Natl Acad Sci USA, 2000. 97(2): p. 835-40.
25. Small-Howard, A., 2009, Detection of fibrin and fibrinogen degradation products and associated methods of production and use for the detection and monitoring of cancer New York Blood Center,
26. Kerber, A., et al., The new DR-70 immunoassay detects cancer of the gastrointestinal tract: a validation study. Aliment Pharmacol Ther, 2004. 20(9): p. 983-7.
27. Ward, D.G., et al., Detection of pancreatic adenocarcinoma using circulating fragments of fibrinogen. Eur J Gastroenterol Hepatol, 2010. 22(11): p. 1358-63.
28. Wu, D., et al., Clinical performance of the AMDL DR-70 immunoassay kit for cancer detection. J Immunoassay, 1998. 19(1): p. 63-72.
29. Hoelz, D.J., R.J. Hickey, and R.H. Malkas, 2006, csPCNA isoform modifications and uses thereof
30. Stoimenov, I. and T. Helleday, PCNA on the crossroad of cancer. Biochem Soc Trans, 2009. 37(Pt 3): p. 605-13.
31. Czyzewska, J., et al., Immunohistochemical evaluation of Ki-67, PCNA and MCM2 proteins proliferation index (PI) in advanced gastric cancer. Folia Histochem Cytobiol, 2009. 47(2): p. 289-96.
32. Venturi, A., et al., Human hepatocellular carcinoma expresses specific PCNA isoforms: an in vivo and in vitro evaluation. Lab Invest, 2008. 88(9): p. 995-1007.
33. Simionescu, C., et al., P53 and PCNA immunoexpression in endometrial carcinomas. Rom J Morphol Embryol, 2006. 47(2): p. 137-41.
34. Mun, K.S., et al., Proliferating cell nuclear antigen (PCNA) activity in hepatocellular carcinoma, benign peri-neoplastic and normal liver. Malays J Pathol, 2006. 28(2): p. 73-7.
35. Malkas, L.H., et al., A cancer-associated PCNA expressed in breast cancer has implications as a potential biomarker. Proc Natl Acad Sci U S A, 2006. 103(51): p. 19472-7.
36. Lyzogubov, V., et al., Immunohistochemical analysis of Ki-67, PCNA and S6K1/2 expression in human breast cancer. Exp Oncol, 2005. 27(2): p. 141-4.
37. Proniewska-Skretek, E., et al., Expression of PCNA and Ki-67 in posterior uveal melanomas in adults. Rocz Akad Med Bialymst, 2004. 49 Suppl 1: p. 79-81.
38. Kushlinskii, N.E., et al., Expression of biomolecular markers (Ki-67, PCNA, Bcl-2, BAX, BclX, VEGF) in breast tumors. Bull Exp Biol Med, 2004. 137(2): p. 182-5.
39. Czyzewska, J., et al., Evaluation of proliferating markers Ki-67, PCNA in gastric cancers. Rocz Akad Med Bialymst, 2004. 49 Suppl 1: p. 64-6.
40. Bantis, A., et al., Expression of p120, Ki-67 and PCNA as proliferation biomarkers in imprint smears of prostate carcinoma and their prognostic value. Cytopathology, 2004. 15(1): p. 25-31.
41. Hickey, R.J., R.H. Malkas, and L. Schnaper, 2006, csPCNA isoform antibodies and uses thereof CS-KEYS, INC,
42. Loveday, R.L.H. and J.H. Greenman, 2008, Cancer Screening Test, US 2008/0305558 A1
43. Kopreski, M.S.L.V.N.J. and C.D.E.C.M.D. Gocke, 2006, Method for detection of hTR and hTERT telomerase-associated RNA in plasma or serum, US 2006/0204956 A1
44. Qureshi, S.A., M.U. Bashir, and A. Yaqinuddin, Utility of DNA methylation markers for diagnosing cancer. Int J Surg, 2010. 8(3): p. 194-8.
45. Deng, D., Z. Liu, and Y. Du, Epigenetic alterations as cancer diagnostic, prognostic, and predictive biomarkers. Adv Genet, 2010. 71: p. 125-76.
46. Ahmed, H., Promoter Methylation in Prostate Cancer and its Application for the Early Detection of Prostate Cancer Using Serum and Urine Samples. Biomark Cancer, 2010. 2010(2): p. 17-33.
47. O'Day, E. and A. Lal, MicroRNAs and their target gene networks in breast cancer. Breast Cancer Res, 2010. 12(2): p. 201.
48. Lin, P.Y., S.L. Yu, and P.C. Yang, MicroRNA in lung cancer. Br J Cancer, 2010. 103(8): p. 1144-8.
49. Rohlff, C.O.B.L.T.F.M.P.A.O.O.O.X.R.Y. and A.O.B.L.T.F.M.P.A.O.O.O.X.R.Y. Stamps, 2009, EPHRIN TYPE-A RECEPTOR 10 PROTEIN, WO 2009/087462 A2
50. Petroziello, J.M.K.W.A., C.-L.S.W.A. Law, and A.F.M.I.W.A. Wahl, 2007, Sga-1 m, a cancer associated antigen, and uses thereof, US 2007/0128593 A1
51. Farnham, P.J.M.W.I., C.R.G.R.M.I. Graveel, and S.R.M.W.I. Harkins-Perry, 2007, Liver tumor marker sequences, US 2007/0042420 A1
52. Macina, R.A.S.J.C.A., et al., 2006, Compositions, splice variants and methods relating to cancer specific genes and proteins, US 2006/0078913 A1
53. Macina, R.A.S.J.C.A., et al., 2006, Composition splice variants and methods relating to cancer specific genes and proteins, US 2006/0160090 A1
54. Macina, R.A.S.J.C.A., et al., 2005, Compositions, splice variants and methods relating to cancer specific genes and proteins, US 2005/0272067 A1
55. O'Brien, T.J.L.R.A.R. and H.K. Tanimoto, 2004, TADG-15: an extracellular serine protease overexpressed in carcinomas, US 2004/0086910 A1
56. Macina, R.A.C.A.S.J.C.A., et al., 2004, COMPOSITIONS, SPLICE VARIANTS AND METHODS RELATING TO CANCER SPECIFIC GENES AND PROTEINS, WO 2004/092338 A2
57. Farnham, P.J.M.W.I., C.R.G.R.M.I. Graveel, and S.R.M.W.I. Harkins-Perry, 2004, Liver tumor marker sequences, US 2004/0086916 A1
58. Rosen, C.A.L.M.D., S.M.O.M.D. Ruben, and S.C.R.M.D. Barash, 2003, Nucleic acids, proteins, and antibodies, US 2003/0108907 A1
59. Petroziello, J.M.N.E.n.S.A.K.W.A., C.-L.F.A.N.S.W.A. Law, and A.F.E.M.W.M.I.W.A. Wahl, 2003, SGA-1M, A CANCER ASSOCIATED ANTIGEN, AND USES THEREOF, WO 2003/065873 A2
60. Mack, D.M.A.M.P.C.A., K.C.P.T.S.F.C.A. Gish, and K.E.J.S.R.C.C.A. Wilson, 2002, METHODS OF DIAGNOSIS OF CANCER, COMPOSITIONS, AND METHODS OF SCREENING FOR CANCER MODULATORS, WO 2002/016939 A2
61. Hevezi, P.S.F.C.A., et al., 2002, Novel methods of diagnosis of prostate cancer and/or breast cancer, compositions, and methods of screening for prostate cancer and /or breast cancer modulators, US 2002/0068036 A1
62. O'Brien, T.J. and H. Tanimoto, 2001, TADG-15: AN EXTRACELLULAR SERINE PROTEASE OVEREXPRESSED IN CARCINOMAS, WO 2001/029056 A1
63. Puscas, I., et al., 1998, RAPID METHOD OF CANCER DIAGNOSIS, WO 1998/041649 A2
64. Mascaux, C., et al., Early detection and screening of lung cancer. Expert Review of Molecular Diagnostics, 2010. 10(6): p. 799-815.
65. Molina, R., et al., Tumor markers (CEA, CA 125, CYFRA 21-1, SCC and NSE) in patients with non-small cell lung cancer as an aid in histological diagnosis and prognosis. Comparison with the main clinical and pathological prognostic factors. Tumour Biol, 2003. 24(4): p. 209-18.
66. Pavicevic, R., et al., CYFRA 21-1 in non-small cell lung cancerstandardisation and application during diagnosis. Coll Antropol, 2008. 32(2): p. 485-98.
67. Huhtinen, K., et al., Serum HE4 concentration differentiates malignant ovarian tumours from ovarian endometriotic cysts. Br J Cancer, 2009. 100(8): p. 1315-9.
68. Hellstrom, I., et al., The HE4 (WFDC2) protein is a biomarker for ovarian carcinoma. Cancer Res, 2003. 63(13): p. 3695-700.
69. Shirodkar, S.P. and V.B. Lokeshwar, Potential new urinary markers in the early detection of bladder cancer. Curr Opin Urol, 2009. 19(5): p. 488-93.
70. Vrooman, O.P. and J.A. Witjes, Urinary markers in bladder cancer. Eur Urol, 2008. 53(5): p. 909-16.
71. Volpe, A., et al., Bladder tumor markers: a review of the literature. Int J Biol Markers, 2008. 23(4): p. 249-61.
72. Shariat, S.F., J.A. Karam, and S.P. Lerner, Molecular markers in bladder cancer. Curr Opin Urol, 2008. 18(1): p. 1-8.
73. Kapila, K., et al., Could nuclear matrix protein 22 (NMP22) play a role with urine cytology in screening for bladder cancer?--experience at Kuwait University. Cytopathology, 2008. 19(6): p. 369-74.
74. Morrissey, J.J., et al., Urinary biomarkers for the early diagnosis of kidney cancer. Mayo Clin Proc, 2010. 85(5): p. 413-21.
75. Walgenbach-Brunagel, G., et al., The use of a colon cancer associated nuclear antigen CCSA-2 for the blood based detection of colon cancer. J Cell Biochem, 2008. 104(1): p. 286-94.
76. Leman, E.S., et al., Evaluation of colon cancer-specific antigen 2 as a potential serum marker for colorectal cancer. Clin Cancer Res, 2008. 14(5): p. 1349-54.
77. Leman, E.S., et al., Initial analyses of colon cancer-specific antigen (CCSA)-3 and CCSA-4 as colorectal cancer-associated serum markers. Cancer Res, 2007. 67(12): p. 5600-5.
78. Gold, D.V., et al., PAM4-reactive MUC1 is a biomarker for early pancreatic adenocarcinoma. Clin Cancer Res, 2007. 13(24): p. 7380-7.
79. Gold, D.V., et al., Characterization of monoclonal antibody PAM4 reactive with a pancreatic cancer mucin. Int J Cancer, 1994. 57(2): p. 204-10.
80. Gold, D.V., et al., New MUC1 serum immunoassay differentiates pancreatic cancer from pancreatitis. J Clin Oncol, 2006. 24(2): p. 252-8.
81. Schilling, D., et al., The Prostate Cancer gene 3 assay: indications for use in clinical practice. BJU Int, 2010. 105(4): p. 452-5.
82. Groskopf, J., et al., APTIMA PCA3 molecular urine test: development of a method to aid in the diagnosis of prostate cancer. Clin Chem, 2006. 52(6): p. 1089-95.
83. de Kok, J.B., et al., DD3(PCA3), a very sensitive and specific marker to detect prostate tumors. Cancer Res, 2002. 62(9): p. 2695-8.
84. Mao, Y., et al., Golgi protein 73 (GOLPH2) is a valuable serum marker for hepatocellular carcinoma. Gut, 2010. 59(12): p. 1687-93.
85. Marrero, J.A., et al., GP73, a resident Golgi glycoprotein, is a novel serum marker for hepatocellular carcinoma. J Hepatol, 2005. 43(6): p. 1007-12.
86. Rollins, G., PSA Testing: Yes, No, Maybe. Clin Lab News, 2009. 35(6): p. 1, 3-5.
87. Smith, D.S., P.A. Humphrey, and W.J. Catalona, The early detection of prostate carcinoma with prostate specific antigen: the Washington University experience. Cancer, 1997. 80(9): p. 1852-6.
88. Schroder, F.H., et al., Screening and prostate-cancer mortality in a randomized European study. N Engl J Med, 2009. 360(13): p. 1320-8.
89. Elmore, J.G., et al., Screening for Breast Cancer. JAMA, 2005. 293(10): p. 1245-1256.
90. Subramanian, S., G. Bobashev, and R.J. Morris, Modeling the cost-effectiveness of colorectal cancer screening: policy guidance based on patient preferences and compliance. Cancer Epidemiol Biomarkers Prev, 2009. 18(7): p. 1971-8.
91. Yurkovetsky, Z., et al., Development of a multimarker assay for early detection of ovarian cancer. J Clin Oncol, 2010. 28(13): p. 2159-66.
92. Gnjatic, S., et al., Seromic profiling of ovarian and pancreatic cancer. Proc Natl Acad Sci USA, 2010. 107(11 ): p. 5088-93.
93. Farlow, E.C., et al., Development of a multiplexed tumor-associated autoantibody-based blood test for the detection of non-small cell lung cancer. Clin Cancer Res, 2010. 16(13): p. 3452-62.
94. Donach, M., et al., Combined use of biomarkers for detection of ovarian cancer in high-risk women. Tumour Biol, 2010. 31(3): p. 209-15.
95. Chen, Y., et al., Autoantibodies to tumor-associated antigens combined with abnormal alpha-fetoprotein enhance immunodiagnosis of hepatocellular carcinoma. Cancer Lett, 2010. 289(1): p. 32-9.
96. Chan, C.C., et al., Multiple serological biomarkers for colorectal cancer detection. Int J Cancer, 2010. 126(7): p. 1683-90.
97. Talesa, V.N., et al., Diagnostic potential in prostate cancer of a panel of urinary molecular tumor markers. Cancer Biomark, 2009. 5(6): p. 241-51.
98. Nosov, V., et al., Validation of serum biomarkers for detection of early-stage ovarian cancer. Am J Obstet Gynecol, 2009. 200(6): p. 639 e1-5.
99. Amonkar, S.D., et al., Development and preliminary evaluation of a multivariate index assay for ovarian cancer. PLoS One, 2009. 4(2): p. e4599.
100. Zhong, L., et al., Autoantibodies as potential biomarkers for breast cancer. Breast Cancer Res, 2008. 10(3): p. R40.
101. Ran, Y., et al., Profiling tumor-associated autoantibodies for the detection of colon cancer. Clin Cancer Res, 2008. 14(9): p. 2696-700.
102. Ludwig, N., et al., Pattern of serum autoantibodies allows accurate distinction between a tumor and pathologies of the same organ. Clin Cancer Res, 2008.14(15): p. 4767-74.
103. Clark, J.P., et al., Performance of a single assay for both type III and type VI TMPRSS2:ERG fusions in noninvasive prediction of prostate biopsy outcome. Clin Chem, 2008. 54(12): p. 2007-17.
104. Zheng, Y., et al., A multiparametric panel for ovarian cancer diagnosis, prognosis, and response to chemotherapy. Clin Cancer Res, 2007. 13(23): p. 6984-92.
105. Patz, E.F., Jr., et al., Panel of serum biomarkers for the diagnosis of lung cancer. J Clin Oncol, 2007. 25(35): p. 5578-83.
106. Lopez-Casas, P.P. and L.A. Lopez-Fernandez, Gene-expression profiling in pancreatic cancer. Expert Rev Mol Diagn, 2010. 10(5): p. 591-601.
107. Bertucci, F., et al., Gene expression profiling of inflammatory breast cancer. Cancer, 2010.116(11 Suppl): p. 2783-93.
108. Nannini, M., et al., Gene expression profiling in colorectal cancer using microarray technologies: results and perspectives. Cancer Treat Rev, 2009. 35(3): p. 201-9.
109. Konstantinopoulos, P.A., D. Spentzos, and S.A. Cannistra, Gene-expression profiling in epithelial ovarian cancer. Nat Clin Pract Oncol, 2008. 5(10): p. 577-87.
110. Cheang, M.C., M. van de Rijn, and T.O. Nielsen, Gene expression profiling of breast cancer. Annu Rev Pathol, 2008. 3: p. 67-97.
111. Arslan, N., et al., Use of CA15-3, CEA and prolactin for the primary diagnosis of breast cancer and correlation with the prognostic factors at the time of initial diagnosis. Ann Nucl Med, 2000. 14(5): p. 395-9.
112. Hou, M.F., et al., Evaluation of serum CA27.29, CA15-3 and CEA in patients with breast cancer. Kaohsiung J Med Sci, 1999. 15(9): p. 520-8.
113. Hayes, D.F., V.R. Zurawski, Jr., and D.W. Kufe, Comparison of circulating CA15-3 and carcinoembryonic antigen levels in patients with breast cancer. J Clin Oncol, 1986. 4(10): p. 1542-50.
114. Harris, L., et al., American Society of Clinical Oncology 2007 update of recommendations for the use of tumor markers in breast cancer. J Clin Oncol, 2007. 25(33): p. 5287-312.
115. Duffy, M.J., et al., Tumour markers in colorectal cancer: European Group on Tumour Markers (EGTM) guidelines for clinical use. Eur J Cancer, 2007. 43(9): p. 1348-60.
116. Suh, K.S., et al., Ovarian cancer biomarkers for molecular biosensors and translational medicine. Expert Review of Molecular Diagnostics, 2010. 10(8): p. 1069-1083.
117. Ren, J., et al., Tumor markers for early detection of ovarian cancer. Expert Rev Mol Diagn, 2010. 10(6): p. 787-98.
118. Moore, R.G., et al., A novel multiple marker bioassay utilizing HE4 and CA125 for the prediction of ovarian cancer in patients with a pelvic mass. Gynecol Oncol, 2009.112(1): p. 40-6.
119. Huerta, S., Recent advances in the molecular diagnosis and prognosis of colorectal cancer. Expert Rev Mol Diagn, 2008. 8(3): p. 277-88.
120. Jemal, A., et al., Cancer statistics, 2009. CA Cancer J Clin, 2009. 59(4): p. 225-49.
121. Jemal, A., et al., Cancer Statistics, 2009. CA Cancer J Clin, 2009: p. caac.20006.
122. Wahls, T.L. and I. Peleg, Patient- and system-related barriers for the earlier diagnosis of colorectal cancer. BMC Fam Pract, 2009. 10: p. 65.
123. Smith, B.D., et al., Future of cancer incidence in the United States: burdens upon an aging, changing nation. J Clin Oncol, 2009. 27(17): p. 2758-65.
124. Lodde, M. and Y. Fradet, The detection of genetic markers of bladder cancer in urine and serum. Curr Opin Urol, 2008. 18(5): p. 499-503.

### CLAUSES:

The present invention will now be described with reference to the following clauses:
1. A method of characterizing a carcinoma in a subject, the method comprising the steps of:
   in a first assay, assaying a sample of a bodily fluid derived from the subject for activity of a first biomarker, the first biomarker being specific for carcinoma but not organ-specific, and
   in a second assay, assaying a sample derived from the subject for a second biomarker, the second biomarker being specific for cancer of an organ and other than extracellular PKA.
2. The method of clause 1 wherein the first assay is an assay for PKA (cAMP-dependent protein kinase A) activity, anti-PKA, fibrin, fibrin derivatives or PCNA (caPCNA; proliferating cell nuclear antigen).
3. The method of clause 1 wherein the first biomarker is extracellular PKA.
4. The method of clause 1 wherein the second assay is carried out after the results of the first assay are known.
5. The method of clause 1 wherein the second assay is carried out before the results of the first assay are known.
6. The method of clause 1 wherein the first and second assays are carried out approximately simultaneously.
7. The method of each of clauses 1-6 wherein the first and second assays are carried out using separate aliquots of the same sample of a bodily fluid.
8. The method of each of clauses 1-7 wherein the second biomarker has a specificity of at least 70%.
9. The method of each of clauses 1-8 wherein the first assay comprises the steps of:
   preparing a reaction mixture comprising the previously unfrozen sample, a PKA peptide substrate, a phosphorylation agent, incubating the prepared mixture, and detecting phosphorylated substrate formed in the incubated mixture, and
   comparing the amount of phosphorylated substrate formed in the assay with a reference value, the reference value being the amount of phosphorylated substrate formed in a mixture under equivalent redox conditions for a sample of bodily fluid derived from a population of normal subjects of the same species.
10. The method of clause 9 wherein extracellular PKA derived from a statistically significant population of subjects unafflicted with a carcinoma and extracellular PKA derived from a statistically significant population of subjects afflicted with a carcinoma have significantly different activities for the phosphorylation of the PKA substrate under the assay conditions.
11. The method of clause 10 wherein a ratio of the activity of extracellular PKA derived from the population of subjects unafflicted with a carcinoma to the activity of extracellular PKA derived from the population of subjects afflicted with a carcinoma for the phosphorylation of the PKA substrate is at least about 1.2:1 or less than about 0.8:1, respectively.
12. The method of clause 10 wherein a ratio of the activity of extracellular PKA derived from the population of subjects unafflicted with a carcinoma to the activity of extracellular PKA derived from the population of subjects afflicted with a carcinoma for the phosphorylation of the PKA substrate is at least about 1.5:1 or less than 0.7:1, respectively.
13. The method of clause 10 wherein a ratio of the activity of extracellular PKA derived from the population of subjects unafflicted with a carcinoma to the activity of extracellular PKA derived from the population of subjects afflicted with a carcinoma for the phosphorylation of the PKA substrate is at least about 1.75:1 or less than about 0.6:1, respectively.
14. The method of clause 10 wherein a ratio of the activity of extracellular PKA derived from the population of subjects unafflicted with a carcinoma to the activity of extracellular PKA derived from the population of subjects afflicted with a carcinoma for the phosphorylation of the PKA substrate is at least about 2:1 or less than 0.5:1, respectively.
15. The method of clause 10 wherein a ratio of the activity of extracellular PKA derived from the population of subjects unafflicted with a carcinoma to the activity of extracellular PKA derived from the population of subjects afflicted with a carcinoma for the phosphorylation of the PKA substrate is at least about 2.25:1 or less than about 0.4:1, respectively.
16. The method of clause 10 wherein a ratio of the activity of extracellular PKA derived from the population of subjects unafflicted with a carcinoma to the activity of extracellular PKA derived from the population of subjects afflicted with a carcinoma for the phosphorylation of the PKA substrate is at least about 2.5:1 or less than about 0.3:1, respectively.
17. The method of clause 10 wherein a ratio of the activity of extracellular PKA derived from the population of subjects unafflicted with a carcinoma to the activity of extracellular PKA derived from the population of subjects afflicted with a carcinoma for the phosphorylation of the PKA substrate is at least about 2.75:1 or less than about 0.25:1, respectively.
18. The method of clause 8 wherein a ratio of the activity of extracellular PKA derived from the population of subjects unafflicted with a carcinoma to the activity of extracellular PKA derived from the population of subjects afflicted with a carcinoma for the phosphorylation of the PKA substrate is at least about 3:1 or less than 0.2:1, respectively.
19. The method of any of clauses 10-18 wherein preparing the reaction mixture comprises treating the sample with a reductant 2-mercaptoethanol between the concentrations of 5 µM and 500 mM.
20. The method of any of clauses 10-18 wherein preparing the reaction mixture comprises treating the sample with a reductant dithiothreitol between the concentrations of 5 µM and 100 mM.
21. The method of any of clauses 1-12 wherein preparing the reaction mixture comprises treating the sample with a reductant dithioerythritol between the concentrations of 5 µM and 100 mM.
22. The method of any of clauses 10-18 wherein preparing the reaction mixture comprises treating the sample with an oxidizing agent.
23. The method of any of clauses 10-18 wherein the preparing the reaction mixture comprises treating the sample with an oxidant diamide between the concentrations of 5 µM and 50 mM.
24. The method of any of clauses 10-18 wherein the preparing the reaction mixture comprises treating the sample with an oxidant hydrogen peroxide between the concentrations of 1 µM and 500 mM.
25. The method of any of clauses 10-24 wherein phosphorylated substrate formed in the incubated mixture is detected by a method not requiring the use of radioactive elements.
26. The method of any of clauses 10-25 wherein the first assay comprises the steps of:
   incubating a mixture comprising a sample of a bodily fluid derived from the subject, a PKA peptide substrate, a phosphorylation agent, and a reducing agent, the mixture having an oxidation reduction potential value that is less than -110 mV or greater than -20 mV, and
   detecting phosphorylated substrate formed in the incubated mixture.
27. The method of any of clauses 10-26 wherein the first assay comprises the steps of:
   incubating a mixture comprising the sample, a PKA peptide substrate, a phosphorylation agent, and a reducing agent, the mixture having an oxidation reduction potential value that is less than -110 mV and greater than -20 mV, and
   detecting phosphorylated substrate formed in the incubated mixture.
28. The method of any clauses 1-27 wherein the carcinoma is selected from the group consisting of lung, colon, pancreatic, ovarian, bladder, and prostate cancer.
29. The method of any of clauses 1-28 wherein the bodily fluid is peripheral blood, whole blood, serum, plasma, ascites, urine, cerebrospinal fluid (CSF), sputum, saliva, bone marrow, synovial fluid, aqueous humor, cerumen, broncheoalveolar lavage fluid, semen, prostatic fluid, cowper's fluid or pre-ejaculatory fluid, sweat, fecal matter, tears, cyst fluid, pleural and peritoneal fluid, breath condensates, nipple aspirate, lymph, chyme, chyle, bile, intestinal fluid, pus, sebum, vomit, mucosal secretion, stool water, pancreatic juice, lavage fluids from sinus cavities, or bronchopulmonary aspirates.
30. The method of any of clauses 1-28 wherein the bodily fluid is peripheral blood, serum, plasma, ascites, urine, cerebrospinal fluid (CSF), sputum, saliva, bone marrow, synovial fluid, aqueous humor, cerumen, broncheoalveolar lavage fluid, semen, prostatic fluid, cowper's fluid or pre-ejaculatory fluid, sweat, fecal matter, tears, cyst fluid, pleural and peritoneal fluid, lymph, chyme, chyle, bile, intestinal fluid, pus, sebum, vomit, mucosal secretion, stool water, pancreatic juice, lavage fluids from sinus cavities, or bronchopulmonary aspirates.
31. The method of any of clauses 1-28 wherein the bodily fluid is selected from among whole blood, sputum, serum, plasma, urine, cerebrospinal fluid, nipple aspirate, saliva, fine needle aspirate, and combinations thereof.
32. The method of any of clauses 1-28 wherein the bodily fluid is serum or urine.
33. The method of any of clauses 1-32 wherein the second biomarker is selected from PSA, PCA3, BTA, NMP-22, ADFP, AQP1, CA19-9, PAM-4, CA125, HE4, CYFRA21-1, GP73, CCSA-2, CCSA-3, CCSA-4, anti-PKA CEA, CA15-3, CA 27.29TIMP-1, MMP-1, MMP-2, MMP-3, MMP-9, a KLK, EGFR, IL-6, IL-6R, or VEGF, extracellular Her-2, sClusterin, P-cadherin, FA-2, mammaglobin, BARD-1, filamin-A, or osteopontin, dentin sialophosphoprotein (DSPP), early prostate cancer antigens (EPCAs), prostate specific membrane antigen (PSMA), prostate secretory protein (PSP), alpha methyl-CoA racemase, chromogranin A, uPA, or uPAR. TGF-beta, IGFBP-2, IGFBP-3 and combinations thereof.
34. The method of any clause 1-32 wherein the first biomarker is selected from the group consisting of p53 autoantibodies, CCL2 autoantibodies, PKA autoantibodies, prostatome autoantibodies, non-organ specific tumor-related methylated DNA, non-organ specific tumor-related miRNA, non-organ specific tumor-related circulating nucleic acid biomarkers and combinations thereof.
35. The method of any clause 1-32 wherein the second biomarker is selected from the group consisting of AMACR autoantibodies, MUC autoantibodies, organ-specific tumor-related methylated DNA, organ-specific tumor-related miRNA, organ-specific tumor-related circulating nucleic acid biomarker, and combinations thereof.

## Claims

1. A method of characterizing a carcinoma in a subject, the method comprising the steps of:
in a first assay, incubating a sample of a bodily fluid derived from the subject for activity of a first biomarker, the first biomarker being specific for carcinoma but not organ-specific; and
in a second assay, assaying a sample derived from the subject for a second biomarker, the second biomarker being specific for cancer of an organ and other than extracellular PKA.

2. The method of Claim 1 wherein the first biomarker is extracellular PKA.

3. The method of Claim 1 or 2 wherein the first and second assays are carried out using separate aliquots of the same sample of a bodily fluid.

4. The method of any one of Claims 1 to 3 wherein the second biomarker has a specificity of at least 70%.

5. The method of any one of Claims 1 to 4 wherein the first assay comprises the steps of:
preincubating a sample for at least 30 minutes,
preparing a reaction mixture comprising the preincubated sample, a PKA peptide substrate, a phosphorylation agent, incubating the prepared mixture, and detecting phosphorylated substrate formed in the incubated mixture, and
comparing the amount of phosphorylated substrate formed in the assay with a reference value, the reference value being the amount of phosphorylated substrate formed in a mixture under equivalent redox conditions for a sample of bodily fluid derived from a population of normal subjects of the same species.

6. The method of Claim 5 wherein extracellular PKA derived from a statistically significant population of subjects unafflicted with a carcinoma and extracellular PKA derived from a statistically significant population of subjects afflicted with a carcinoma have significant different activities for the phosphorylation of the PKA substrate under the assay conditions.

7. The method of Claim 6 wherein a ratio of the activity of extracellular PKA derived from the population of subjects unafflicted with a carcinoma to the activity of extracellular PKA derived from the population of subjects afflicted with a carcinoma for the phosphorylation of the PKA substrate is at least about 1.2:1 or less than about 0.8:1, respectively.

8. The method of Claim 4 wherein a ratiwo of the activity of extracellular PKA derived from the population of subjects unafflicted with a carcinoma to the activity of extracellular PKA derived from the population of subjects afflicted with a carcinoma for the phosphorylation of the PKA substrate is at least about 3:1 or less than 0.2:1, respectively.

9. The method of any one of Claims 6 to 8 wherein preparing the reaction mixture comprises treating the sample with an oxidizing agent or a reductant for at least 30 minutes.

10. The method of any one of Claims 6 to 9 wherein preparing the reaction mixture comprises preincubating the sample with:
a reductant 2-mercaptoethanol between the concentrations of 5 µM and 500 mM;
a reductant dithiothreitol between the concentrations of 5 µM and 100 mM; or
a reductant dithioerythritol between the concentrations of 5 µM and 100 mM.

11. The method of any one of Claims 6 to 8 wherein the first assay comprises the steps of:
preincubating a sample of a bodily fluid for at least 30 minutes with a reducing agent;
adding the preincubated sample to a PKA peptide substrate, and a phosphorylation agent, the mixture having an oxidation reduction potential value that is less than -110 mV or greater than -20 mV; and
detecting phosphorylated substrate formed in the incubated mixture.

12. The method of any one of Claims 6 to 11 wherein the first assay comprises the steps of:
preincubating a sample of a bodily fluid for at least 30 minutes with an oxidising agent;
adding the preincubated sample to a PKA peptide substrate, and a phosphorylation agent, the mixture having an oxidation reduction potential value that is less than -110 mV or greater than -20 mV; and
detecting phosphorylated substrate formed in the incubated mixture.

13. The method of any one of Claims 1 to 12 wherein the carcinoma is selected from the group consisting of lung, colon, pancreatic, ovarian, bladder, and prostate cancer.

14. The method of any one of Claims 1 to 13 wherein the bodily fluid is plasma, serum or urine.

15. The method of any one of Claims 1 to 13 wherein the second biomarker is selected from PSA, PCA3, BTA, NMP-22, ADFP, AQP1, CA19-9, PAM-4, CA125, HE4, CYFRA21-1, GP73, CCSA-2, CCSA-3, CCSA-4, anti-PKA CEA, CA15-3, CA 27.29 TIMP-1, MMP-1, MMP-2, MMP-3, MMP-9, a KLK, EGFR, IL-6, IL-6R or VEGF, extracellular Her-2, sClusterin, P-cadherin, FA-2, mammaglobin, BARD-1, filamin-A, or osteopontin, dentin sialophosphoprotein (DSPP), early prostate cancer antigens (EPCAs), prostate specific membrane antigen (PSMA), prostate secretory protein (PSP), alpha methyl-CoA racemase, chromogranin A, uPA, or uPAR, and combinations thereof.
